# EUROPEAN PATENT SPECIFICATION

(11) **EP 2 167 038 B1**
(45) Date of publication and mention of the grant of the patent: **25.04.2018**
(21) Application number: 08771043.0
(22) Date of filing: 13.06.2008
(51) Int. Cl.: A61K 9/06, A61K 9/00, A61K 38/00

(54) **USE OF TACI-IG FUSION PROTEIN SUCH AS ATACICEPT FOR THE MANUFACTURE OF A MEDICAMENT FOR TREATING LUPUS ERYTHEMATOSUS**
DOSIERMETHODEN ZUR BEHANDLUNG VON AUTOIMMUNKRANKHEITEN MIT EINEM TACI-IG-FUSIONSPROTEIN WIE ATACICEPT
EMPLOI DE PROTÉINE DE FUSION DE TACI-IG TEL QUE DE L'ATACÈPTE POUR LA PRODUCTION D'UN MÉDICAMENT POUR TRAITER LUPUS ÉRYTHÉMATEUX

(30) Priority: 13.06.2007 US 943618 P; 28.01.2008 US 24031
(43) Date of publication of application: 31.03.2010
(73) Proprietor: Zymogenetics, Inc., Seattle, WA 98102 (US); ARES TRADING S.A., 1170 Aubonne (CH)
(72) Inventor: BUSBY, Sharon J., Seattle, Washington 98103 (US); GROSS, Jane A., Seattle, Washington 98115 (US); VISICH, Jennifer, San Francisco, CA 94080 (US); NESTOROV, Ivan, Issaguah, Washington 98029 (US); MUNAFO, Alain, CH-1180 Tartegnin (CH); PAPASOULIOTIS, Orestis, CH-1208 Geneva (CH); PENA ROSSI, Claudia, CH-1205 Geneva (CH)
(74) Representative: Griffin, Philippa Jane
(86) International application number: PCT/US2008/066945
(87) International publication number: WO 2008/157369

(56) References cited:
- US-A1- 2007 071 760
- STOHL ET AL: "B cell depletion therapy in systemic rheumatic diseases: Different strokes for different folks?" CLINICAL IMMUNOLOGY, ACADEMIC PRESS, US, vol. 121, no. 1, 1 October 2006 (2006-10-01), pages 1-12, XP005653909 ISSN: 1521-6616
- BELL E: "TNF-R homologues in autoimmune disease" IMMUNOLOGY TODAY, ELSEVIER PUBLICATIONS, CAMBRIDGE, GB, vol. 21, no. 6, 1 June 2000 (2000-06-01), page 253, XP004200084 ISSN: 0167-5699

## Description

### FIELD OF THE INVENTION

In various embodiments, the present invention relates to methods and compositions for the treatment of autoimmune diseases or disorders of the immune system, comprising administering a TACI-Ig fusion protein such as atacicept using a particular dosage regime which maximizes the blocking of functions of the ligands of the TNF family.

### BACKGROUND OF THE INVENTION

### The BlyS Ligand / Receptor Family

Three receptors, TACI (transmembrane activator or Calcium-Modulating Cyclophylin Ligand-interactor), BCMA (B-cell maturation antigen) and BAFF-R (receptor for B-cell activating factor, belonging to the TNF family), have been identified that have unique binding affinities for the two growth factors BLyS (B-lymphocyte stimulator) and APRIL (a proliferationinducing ligand) (Marsters et al. Curr Biol2000; 10(13): 785-788; Thompson et al. Science 200 1 ; 293:2 1 08-2 1 11). TACI and BCMA bind both BLyS and APRIL, while BAFF-R appears capable of binding only BLyS with high affinity (Marsters et al. Curr Biol2000; 10(13):785-788; Thompson et al. Science 2001; 293:21 08-21 11 .). As a result, BLyS is able to signal through all three receptors, while APRIL only appears capable of signaling through TACI and BCMA. In addition, circulating heterotrimer complexes of BLyS and APRIL (groupings of three proteins, containing one or two copies each of BLyS and APRIL) have been identified in serum samples taken from patients with systemic immune-based rheumatic diseases, and have been shown to induce B-cell proliferation in vitro (Roschke et al. J Immunol 2002; 169: 4314-4321). Amongst the Ig-fusion proteins for all three receptors, only TACI-Fc5, such as atacicept, was able to block the biological activity of the heterotrimeric complexes (Roschke et al. J Immunol 2002; 169: 43 14-4321).

BLyS and APRIL are potent stimulators of B-cell maturation, proliferation and survival (Gross et al. Nature 2000; 404: 995-999. Gross et al. Immunity 2001; 15(2): 289-302. Groom et al. J Clin Invest 2002; 109(1): 59-68). BLyS and APRIL may be necessary for persistence of autoimmune diseases, especially those involving B-cells. Transgenic mice engineered to express high levels of BLyS exhibit immune cell disorders and display symptoms similar to those seen in patients with Systemic Lupus Erythematosus (SLE) (Cheson et al. Revised guidelines for diagnosis and treatment. Blood 1996; 87:4990-4997. Cheema et al. Arthritis Rheum 2001 ; 44(6): 13 13-1 3 19). Similarly, increased levels of BLyS and APRIL have been measured in serum samples taken from SLE patients and other patients with various autoimmune diseases like Rheumatoid Arthritis (Roschke et al. J Immunol 2002; 169:43 14-4321 ; Mariette X., Ann Rheum Dis 2003; 62(2): 168-17 1 ; Hahne et al. J Exp Med 1998; 188(6): 1 185-1 190), extending the association of BLyS and/or APRIL and B-cell mediated diseases from animal models to humans.

### Systemic Lupus Erythematosus

Systemic lupus erythematosus (SLE) is an autoimmune disease clinically characterized by a waxing and waning course and by involvement of multiple organs including skin, kidneys and central nervous system (Kammer G M and Tsokos G C Eds. (1999) Lupus: Molecular and Cellular Pathogenesis 1st Ed, Human Press, N.J.; Lahita R G Ed. (1999) Systemic Lupus Erythromatosus, 3rd Ed, Academic Press, Amsterdam). The overall prevalence of SLE is about one in 2000, and about one in 700 Caucasian women develops SLE during her life time. (Lahita R G (1999) Curr. Opin. Rheumatol. Sep;11(5):352-6). In the United States alone, over half a million people have SLE, and most are women in their childbearing years (Hardin J A (2003) J. Exp. Med. 185:1101-1111).

There is no single criteria to diagnose SLE. The American College of Rheumatology has developed 11 criteria to diagnose SLE, which span the clinical spectrum of SLE in aspects of skin, systemic, and laboratory tests. These criteria include malar rash, discoid rash, sensitivity to sun light, oral ulcers, arthritis, serositis, kidney and central nervous system inflammation, blood alterations, and the presence of antinuclear antibodies. A patient must meet four of these criteria in order to be classified as a SLE patient. (Tan et al. (1982) Arthritis Rheumatol. 25:1271-1277). SLE is usually confirmed by tests including, but not limited to, blood tests to detect anti-nuclear antibodies; blood and urine tests to assess kidney function; complement tests to detect the presence of low levels of complement that are often associated with SLE; a sedimentation rate (ESR) or C-reactive protein (CRP) to measure inflammation levels; X-rays to assess lung damage and EKGs to assess heart damage.

The standard therapy for SLE is administration of the steroid glucocorticoid, a general immune response inhibitor. It can be used to relieve symptoms; however, no cure for SLE is currently available. Low dose p.o. prednisone at a level less than 0.5 mg/kg/day is usually given. Unfortunately, this therapy is insufficient to keep patients in remission, and flaring of the disease is frequent. Flares can be controlled with high dose glucocorticoid via intravenous pulses at 30mg methylprednisolone/kg/day for 3 consecutive days. However, steroid treatment at high dosage can present severe side effects for patients.

These standard treatments are generally nonspecific, are frequently associated with serious side-effects and do not significantly affect the progression of the disease or transition to life threatening kidney complications (lupus nephritis or LN). Consequently, there is a long-felt need in the art to develop new methods for treating SLE.

US 2007/0071760 describes methods for treating B cell malignancies using a TACI-Ig fusion molecule.

Stohl & Looney, Clinical Immunology (2006), Vol. 121(1), pages 1-12, describes B cell depletion therapy in systemic rheumatic diseases.

Bell E., Immunology Today, Vol. 21(6), 1 June 2000, page 253, describes TNF-R homologues in autoimmune disease.

The present disclosure is directed to methods of treating autoimmune diseases. Illustratively, the methods disclosed herein including administering to a patient a composition comprising a human immunoglobulin-constant domain and TACI extracellular domain or a fragment thereof which binds BLyS and/or APRIL.

The disclosure comprises methods of treating autoimmune diseases, including SLE, using a molecule that comprises a fusion of the TACI extracellular domain or any fragment thereof that retains the ability to bind BLyS and/or APRIL, such as atacicept.

The disclosure comprises methods of treating SLE comprising administering to a patient in need thereof an effective amount of a fusion molecule comprising a human immunoglobulin-constant chain and TACI extracellular domain or a fragment of TACI extracellular domain that binds BLyS and/or APRIL. In one embodiment, the fragment of the extracellular domain of TACI comprises one or two cysteine repeat motifs. In another embodiment, the fragment is a fragment comprising amino acids 30-110 of the extracellular domain of TACI. In yet another embodiment, the fragment is a fragment comprising amino acids I-154 of the extracellular domain of TACI (SEQ ID NO: 1).

The disclosure comprises methods of treating SLE by administering to a patient a composition comprising a fusion polypeptide, TACI-Fc5, comprising a human immunoglobulin-constant domain, Fc5, having the sequence set out as SEQ ID NO: 2 and a TACI extracellular domain having the sequence set out as SEQ ID NO: 1.

The disclosure comprises methods of treating SLE by administering to a patient a composition comprising a fusion polypeptide comprising a human immunoglobulin-constant domain with the sequence set out as SEQ ID NO: 2 and a polypeptide which binds BLyS and/or APRIL and which is at least about 50%, at least about 60%, at least about 65%, at least about 70%, at least about 75%, at least about 80%, at least about 85%, at least about 90%, at least about 95% or at least about 99% identical to SEQ ID NO: 1.

Other autoimmune diseases can be treated by the methods of the invention by administering to a patient a fusion polypeptide comprising a human immunoglobulin-constant chain and TACI extracellular domain or a fragment of TACI extracellular domain that binds BlyS and/or APRIL. Such autoimmune diseases include, but are not limited to rheumatoid arthritis (RA), Graves disease, type I and type II diabetes, multiple sclerosis, Sjogren syndrome, scleroderma, glomerulonephritis, transplant rejection, e.g., organ and tissue allograft and xenograft rejection and graft versus host disease.

In one embodiment, the methods of the instant invention comprise administering to a SLE patient atacicept fusion molecule in amounts from about 0.01 mg/kg of patient's body weight to about 25 mg/kg of patient's body weight. The atacicept molecule can be administered repeatedly at predetermined intervals. Illustratively, the molecule can be administered multiple times during predetermined dosing intervals. For example, dosing can be of a relatively lower dose of drug at weekly or every three week intervals. An initial treatment with a Atacicept fusion polypeptide can be followed by administering the polypeptide on a bi-weekly (every other week) or tri-weekly (every third week) basis for at least 2 or 3 more additional weeks, respectively. For example, the polypeptide can be administered on a bi-weekly basis for an additional 2 to 30 weeks. Alternately, the polypeptide may be administered on a weekly or daily basis.

According to the methods of the instant invention, atacicept polypeptide can be administered to a SLE patient subcutaneously, orally, or intravenously and in combination with other medicaments. Such medicaments include, but are not limited to: NSAIDS (nonsteroidal anti-inflammatory drugs) both over the counter and those requiring a prescription such as diclofenac sodium, indomethacin diflunisal and nabumetone; anti-malarials such as hydroxychloroquine sulfate and chloroquine; corticosteroids such as prednisone, hydrocortisone, and methylprednisolone; and immunosuppressives such as azathioprine, cyclophosphamide, methotrexate, cyclosporine, and mycophenolate mofetil, and IVIg, DHEA, and thalidomide.

### BRIEF DESCRIPTION OF THE FIGURES

FIGURE 1 graphically represents the free atacicept concentration plotted versus time in days for subcutaneous administration, a key pharmakinetic measurement. Each line of the graph is a dosage, as shown in the key.
FIGURE 2 graphically represents the atacicept:BLyS complex concentration plotted versus time in days for subcutaneous administration, a key pharmakinetic measurement. Each line of the graph is a dosage, as shown in the key of Figure 1.
FIGURES 3A and 3B graph the biological effects of subcutaneous administration on various immunoglobulin levels and B cell levels.
FIGURE 4 shows the difference in bioavailability as shown by free atacicept measurements for subcutaneous and intravenous administration of atacicept.
FIGURE 5 graphs the relatively similar biological activity seen with the two administration methods, as represented by IgM concentration versus time. See key on Figure 4.

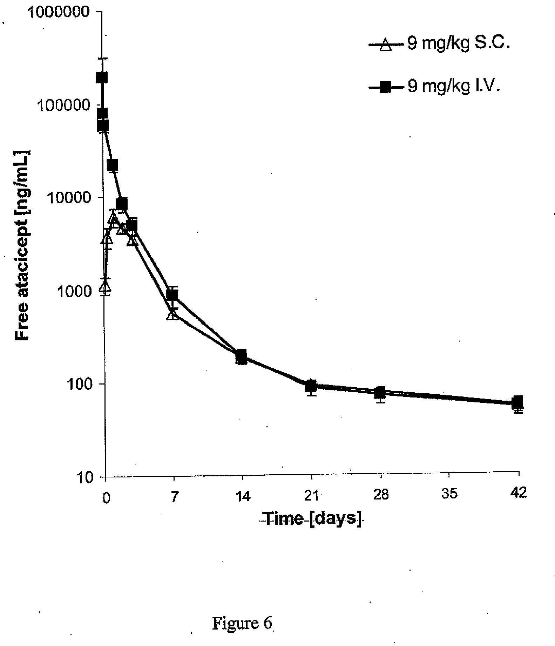

FIGURE 6 graphically shows the similarity between the subcutaneous and intravenous administration methods, as represented by free atacicept versus time.
FIGURE 7 shows the relatively similar target binding curves seen with the two administration methods, as represented by atacicept :BLyS complex versus time. See key on Figure 4.
FIGURE 8 graphically shows how multiple doses yield higher biological activity, as represented by IgM concentration versus time.
FIGURE 9 graphically shows how target binding is higher with multiple doses, as represented by atacicept:BLys complex versus time. See key on Figure 8.
FIGURE 10 graphically represents the free atacicept concentration plotted versus time in days for intravenous administration, a key pharmakinetic measurement. Each line of the graph is a dosage, as shown in the key.
FIGURE 11 graphically represents the atacicept:BLyS complex concentration plotted versus time in days for intravenous administration, a key pharmakinetic measurement. Each line of the graph is a dosage, as shown in the key of Figure 10.

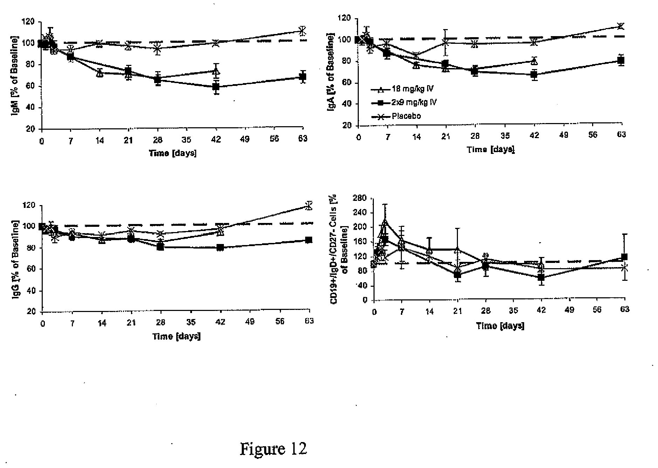

FIGURE 12 is a graphic representation of the biomarker measurements using intravenous administration, specifically immunoglobulin levels and B cell levels.

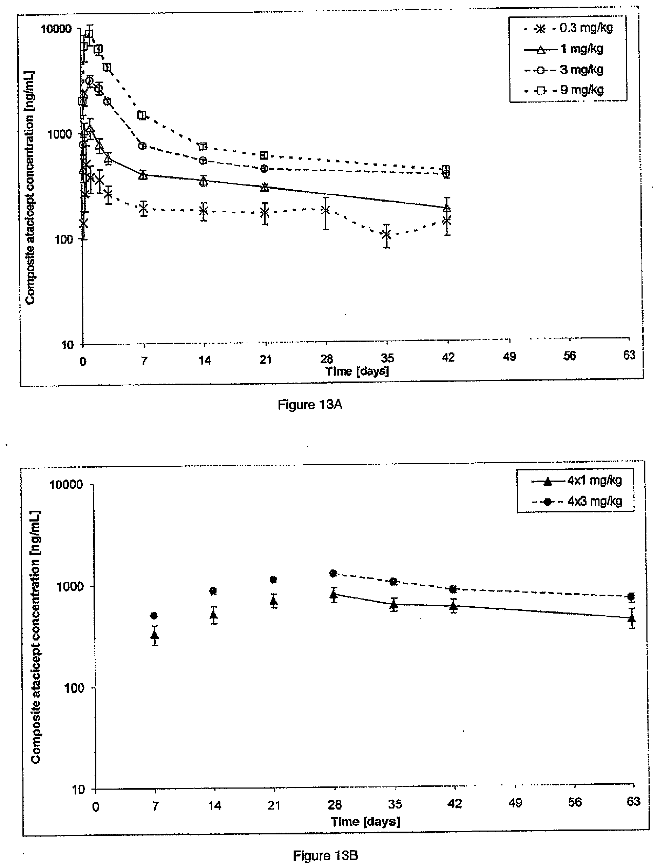

FIGURE 13A and B are graphic representations of the composite atacicept concentration (defined as free atacicept + atacicept-BLyS complex) vs time for subcutaneous administration (Study 1). (A) Single-dose cohorts; (B) Multiple-dose cohorts. Mean ± SE values are presented. Multiple doses were administered at days 0, 7, 14, and 21. Points during dosing are not connected to indicate concentration peaks were not captured between doses.

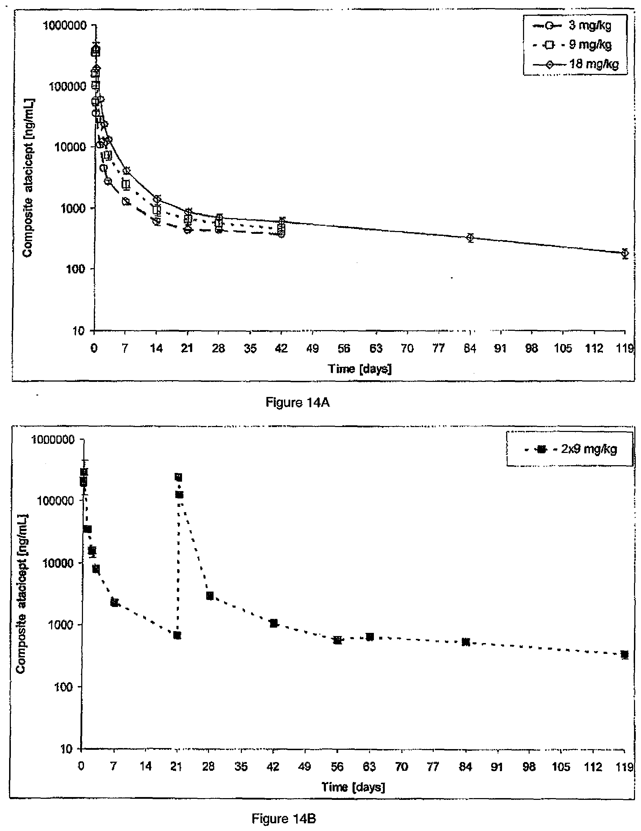

FIGURE 14A and B are graphic representations of the composite atacicept concentration (defined as free atacicept + atacicept-BLyS complex) vs time for intravenous administration (Study 2). (A) Single-dose cohorts; (B) Multiple-dose cohorts. Mean ± SE values are presented. Multiple doses were administered at days 0 and 21.

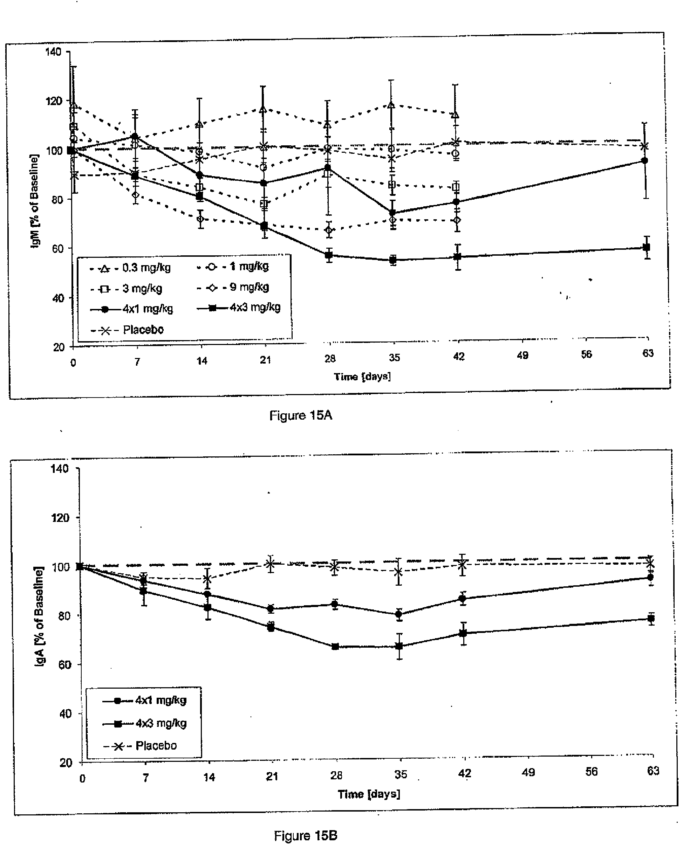

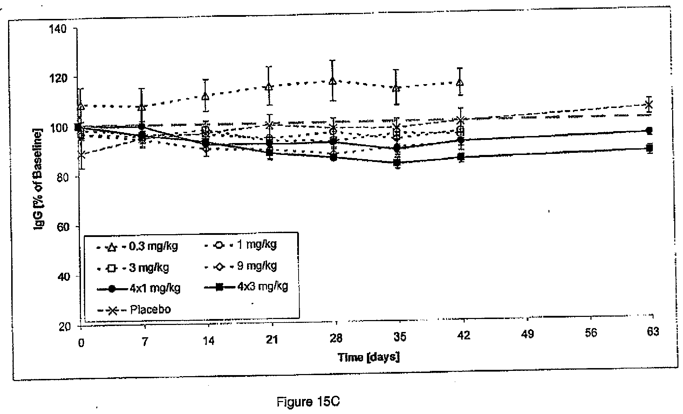

FIGURE 15A, B, and C are immunoglobulin summary profiles in Study 1 (subcutaneous administration) by cohort (% of baseline, mean ± SE). (A) IgM; (B) IgA, (C) IgG. These figures extend the data presented in Figure 3A and 3B.

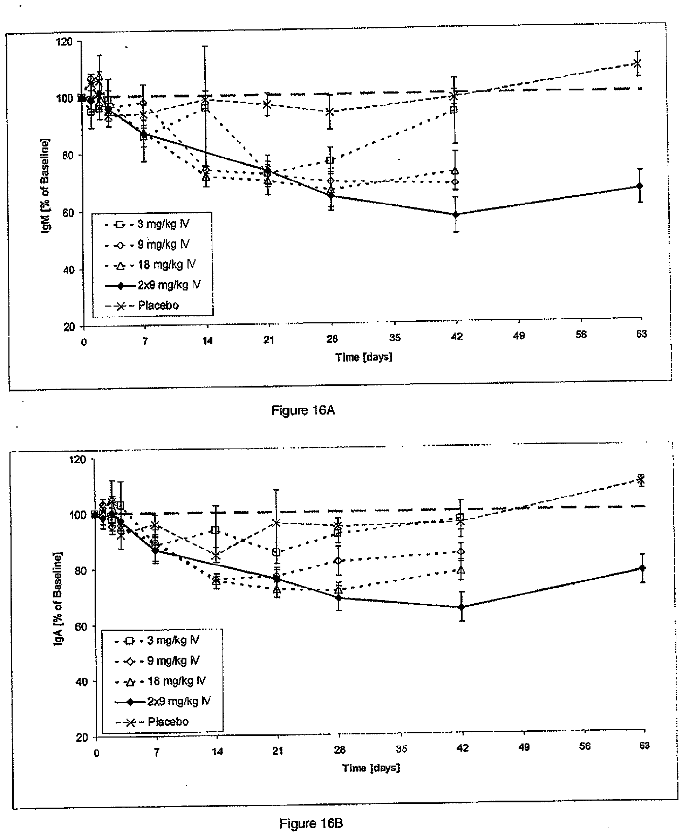

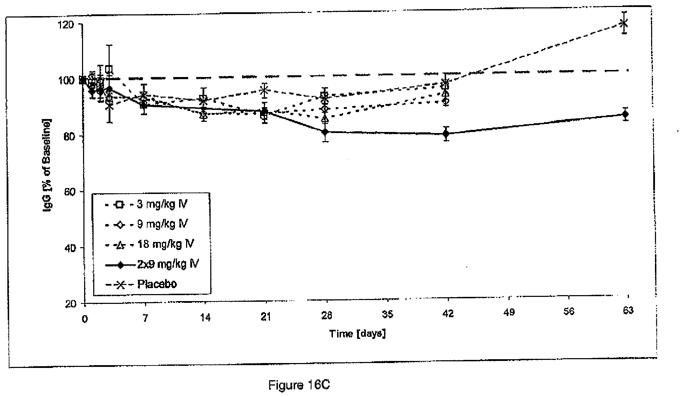

FIGURE 16A, B, and C are immunoglobulin summary profiles in Study 2 (intravenous administration) by cohort (% of baseline, mean ± SE). (A) IgM; (B) IgA, (C) IgG. These figures extend the data presented in Figure 12.

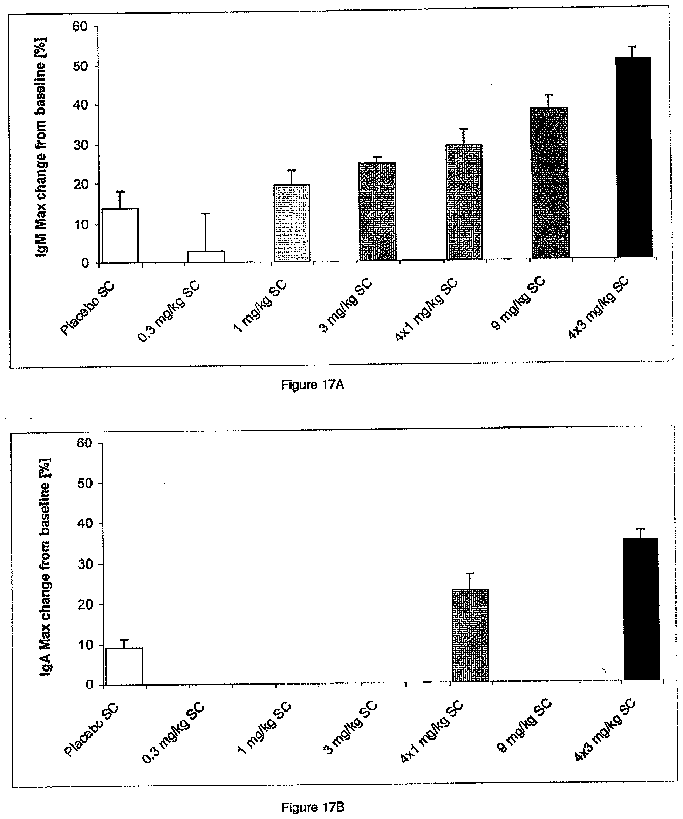

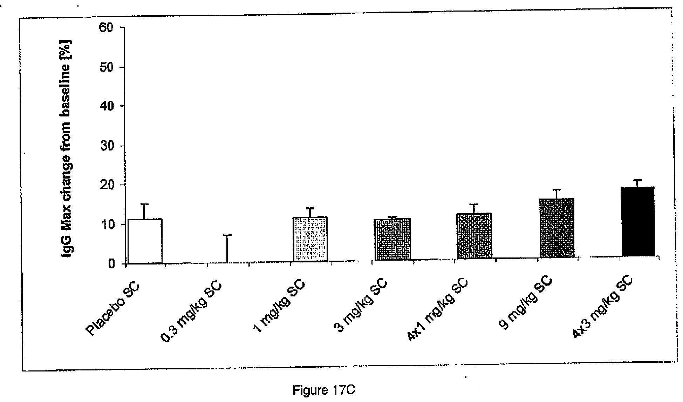

FIGURE 17A, B, and C graphs the relationship between atacicept subcutaneous dose (Study 1) and the observed maximum immunoglobulin response (in % decrease from baseline). Bars represent mean± SE. (A) IgM; (B) IgA, (C) IgG.

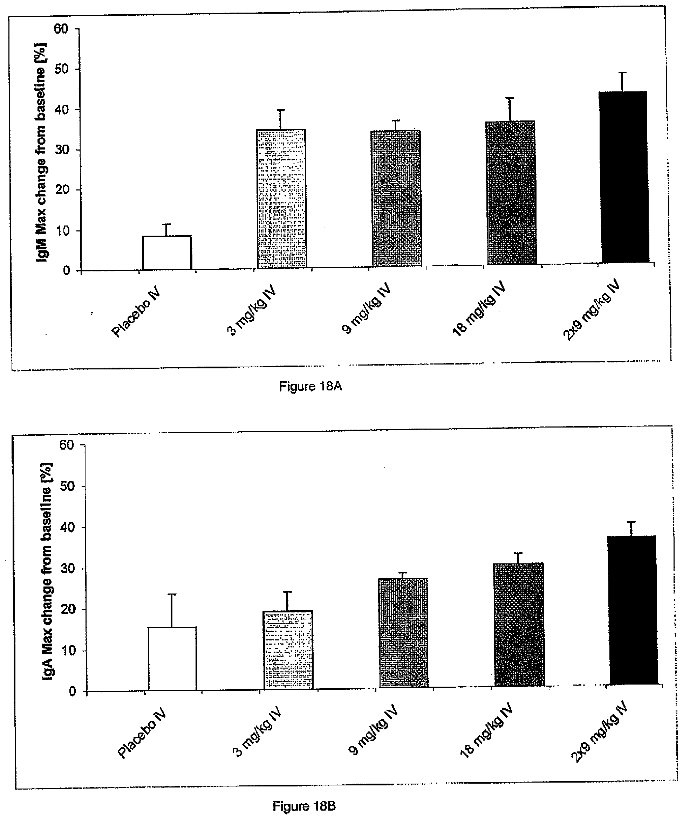

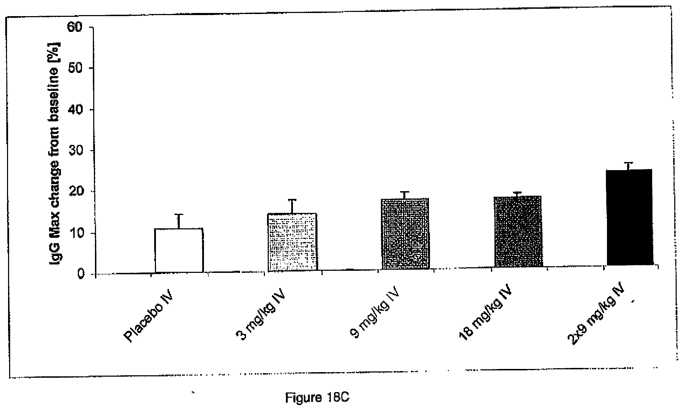

FIGURE 18A, B, and C graphs the relationship between atacicept intravenous dose (Study 2) and the observed maximum immunoglobulin response (in % decrease from baseline). Bars represent mean± SE. (A) IgM; (B) IgA, (C) IgG.
FIGURE 19A and B shows IgM profiles (mean± SE) in the same single dose cohorts of the subcutaneous and intravenous studies. (A) 3 mg/kg; (B) 9 mg/kg.
FIGURE 20A and B shows Atacicept:BLyS complex profiles (mean ± SE) in the same single dose cohorts of the subcutaneous and intravenous studies. (A) 3 mg/kg; (B) 9 mg/kg.

### DETAILED DESCRIPTION OF THE INVENTION

In various embodiments, the instant invention pertains to methods of treating an autoimmune disease in a patient by inhibiting interaction of BlyS and/or APRIL with their receptors. The patient may be a mammal, for example a human. In one embodiment, the methods utilize an inhibitor that comprises: 1) a polypeptide that comprises a domain which is at least partially identical to TACI extracellular domain or a fragment thereof that binds BlyS and/or APRIL; and 2) a human immunoglobulin constant chain. In one embodiment, the methods of the invention utilize a fusion molecule comprising a human immunoglobulin constant chain and any polypeptide with at least about 50%, at least about 60%, at least about 65%, at least about 70%, at least about 75%, at least about 80%, at least about 85%, at least about 90%, at least about 95% or at least about 99% sequence identity to TACI extracellular domain. U.S. Patent Nos. 5,969,102, 6,316,222 and 6,500,428 and U.S. patent applications 091569,245 and 091627,206 (teachings of which are incorporated herein in their entirety by reference) disclose sequences for the extracellular domain of TACI as well as specific fragments of the TACI extracellular domain that interact with TACI ligands, including BlyS and APRIL. One illustrative fragment of the extracellular domain of TACI comprises one or two cysteine repeat motifs. Another illustrative fragment is a fragment comprising amino acids 30-110 of the extracellular domain of TACI or fragments thereof. Yet another illustrative fragment is a fragment comprising amino acids 1-154 of the extracellular domain of TACI (SEQ ID NO: 1) or fragments thereof.

Other fusion molecules useful for the methods of the invention include: a fusion polypeptide between a human immunoglobulin constant chain and the complete TACI extracellular domain or its ortholog or a fusion polypeptide between a human immunoglobulin constant chain and any fragment of the extracellular TACI domain that can bind BlyS and APRIL ligands. Any of the fusion molecules used in the methods of the invention can be referred to as a TACI-Ig fusion molecule.

TACI-Fc5 is one of the TACI-Ig fusion molecules useful for the methods of the invention. TACI-Fc5 is a recombinant fusion polypeptide comprising the extracellular, ligand binding portion of receptor TACI from about amino acid 1 to about amino acid 154 (SEQ ID NO: I) and the modified Fc portion of human IgG, Fc5 (SEQ ID NO: 2). Other TACI-Ig molecules useful for the methods of the instant invention include a fusion molecule comprising polypeptide with SEQ ID NO: 2 and a polypeptide which can bind BlyS and which is at least about 50%, at least about 60%, at least about 65%, at least about 70%, at least about 75%, at least about 80%, at least about 85%, at least about 90%, at least about 95% or at least about 99% identical to SEQ ID NO: 1.

Embodiments of the instant invention comprise methods of using a TACI-Ig fusion molecule for treating SLE. Other autoimmune diseases that can be treated with the methods of the invention include rheumatoid arthritis (RA), Graves disease, type I and type II diabetes, multiple sclerosis, Sjogren syndrome, scleroderma, glomerulonephritis, transplant rejection, e.g., organ and tissue allograft and xenograft rejection, graft versus host disease or any other autoimmune disease that may be treated by decreasing the number of circulating mature B cells and immunoglobulin-secreting cells and soluble immunoglobulins associated with such diseases.

Embodiments also comprise methods of treatment by administering to a patient a fusion molecule comprising a human immunoglobulin-constant domain and a polypeptide comprising any fragment of TACI extracellular domain that can bind BlyS and/or APRIL.

A TACI-Ig fusion molecule can be administered to a patient according to any suitable route of administration, including by not limited to orally, intravenously or subcutaneously.

TACI-Ig formulations useful for the methods of the invention can be prepared and stored as a frozen, sterile, isotonic solution. Such formulations can include other active ingredients and excipients such as, for example, sodium chloride, phosphate buffer and sodium hydroxide or 0-phosphoric acid (pH 6.0). TACI-Ig formulations can be administered to a patient in combination with other medicaments. Such medicaments include but are not limited to NSAIDS (nonsteroidal anti-inflammatory drugs) both over the counter and those requiring a prescription such as diclofenac sodium, indomethacin diflunisal and nabumetone; anti-malarials such as hydroxychloroquine sulfate and chloroquine; corticosteroids such as prednisone, hydrocortisone, and methylprednisolone; and immunosuppressives such as azathioprine, cyclophosphamide, methotrexate, cyclosporine, and mycophenolate mofetil, and IVIg, DHEA, and thalidomide.

Methods of the invention can be used in combination with other methods of treating autoimmune diseases. Such other methods of treatment include, but are not limited to surgery, acupuncture, physical therapy and gene therapy. TACI-Ig formulations can be administered prior, simultaneously or subsequently to other methods of treatment.

TACI-Fc5 has been shown to inhibit BLyS activation of B cell proliferation in vitro. Treatment of mice with TACI-Fc5 results in a partial block in B cell development that has a minimal effect on B cell precursors in the bone marrow and other cell lineages including peripheral blood T cells, monocytes and neutrophils. Transgenic mice engineered to overexpress a soluble form of the TACI receptor in the blood produce fewer mature B cells and show reduced levels of circulating antibody. The TACI-Fc5 transgenic mice had normal numbers of cells in the thymus, bone marrow and mesenteric lymph node. There were no significant differences in T cell populations in the thymus, lymph node and spleen. (Gross et al. Immunity 2001; 15(2): 289-302.)

Further, TACI-Ig can inhibit antigen-specific antibody production in an immune response in mice whether administered during the primary response or the secondary response to an antigen. In these studies, no effect on T cell response to ex vivo antigenic challenge was observed. In an animal model of systemic lupus erythematosus, treatment with TACI-Ig fusion proteins was effective in limiting the onset and progression of the disease. (Gross et al. Nature 2000; 404: 995-999). Similarly, in a mouse model of collagen-induced arthritis, TACI-Ig was able to inhibit the development of collagen-specific antibodies and reduce both the incidence of inflammation and the rate of occurrence of disease. (Gross et al. Immunity 2001; 15(2): 289-302).

A composition comprising a TACI-Ig fusion molecule may be administered to a patient once or may be administered to a patient repeatedly over a period of time. For example, a patient may receive one subcutaneous injection of TACI-Ig molecules after which his or her condition may be monitored. Patients who demonstrate improvement or at least stabilization of their condition may be administered a TACI-Ig fusion molecule repeatedly for an additional period of time. The additional period of time may be from about 2 to about 52 weeks. For example, a patient may be administered three doses of TACI-Ig fusion molecule during a four week interval. Alternately, a patient may be administered seven doses of a TACI-Ig fusion molecule during a twelve week interval. The administration of TACI-Ig molecules to a patient may be daily, bidaily, weekly, bi-weekly, tri-weekly, monthly, bi-monthly, etc.

A TACI-Ig fusion molecule is administered to a patient in amount that is efficient for treating the patient's condition. In one embodiment, the term "treating" in relation a given disease or disorder, includes, but is not limited to, inhibiting the disease or disorder, for example, arresting the development of the disease or disorder; relieving the disease or disorder, for example, causing regression of the disease or disorder; or relieving a condition caused by or resulting from the disease or disorder, for example, relieving, preventing or treating symptoms of the disease or disorder. In another embodiment, the amount may range from about 0.01 mg per 1 kg of patient's body weight to about 20 mg per 1 kg of patient's body weight.

A fusion TACI-Ig molecule may be delivered in any suitable manner. In one embodiment, the molecule is delivered by peritoneal injection. In another embodiment, the peritoneal injection is via subcutaneous injection. In another embodiment, the peritoneal injection is administered into the anterior abdominal wall. When more than one injection is required to administer a dose, the injections can be administered a few centimeters apart and relatively close together in time, for example as close as is reasonably possible. For repeated drug administration, the site of administration on the anterior abdominal wall can be rotated or alternated. Exemplary zones for subcutaneous injection into the anterior abdominal wall include right upper external area, left lower external area, right lower external area, left upper external area, median lower area as well as right and left thighs and upper arms. Alternatively, a TACI-Ig fusion molecule of the instant invention may delivered via intravenous injections or orally in a form of tablets, caplets, liquid compositions or gels, etc.

B cells are currently thought to play an important role in SLE pathogenesis, through both antibody-dependent and antibody-independent mechanisms. In addition to antibody production, B cells secretenumerous cytokines, act as antigen presenting cells, and serve a variety of effectorfunctions. Thus, B cells have emerged as rational targets for drug development in SLE (Browning JL., Nat Rev Drug Discov 2006;5:564-76).

Several B-cell-directed strategies have been proposed as possible therapies for SLE. Some of these strategies are designed to eliminate B cells through the use of Bcell-directed monoclonal antibodies (mAb) (Leandro MJ, Edwards JC, Cambridge GI Ehrenstein MR, Isenberg DA. Arthritis Rheum 2002;46:2673-3; Looney RJ, Anolik JH, Campbell D1 Felgar RE, Young F, Arend LJ, et al., Arthritis Rheum 2004;50:2580-9; Leandro MJ, Cambridge G, Edwards JC, Ehrenstein MR, Isenberg DA., Rheumatology 2005;44:1542-5; Dorner T, Kaufman J, Wegener WA, Teoh N, Goldenberg DM, Burmester GR., Arthritis Res Ther 2006;8:R74). While others interfere with B cell stimulation (Baker KP, Edwards BM, Main SH, Choi GH, Wager RE, Halpern WG, et al. Arthritis Rheum 2003;48:3253-65; Wallace DJ, Lisse J, Stohl W, McKay J, Boling El Merrill JT, et al., American College of Rheumatology Annual Scientific Meeting, 2006; Gross JA, Dillon SR, Mudri S, Johnston J, Littau A, Roque R, et al., Immunity 2001; 15:289-302) or seek to selectively target autoantibody-producing B cells (Alarcon-Segovia D, Tumlin JA, Furie RA, McKay JD, Cardiel, MH, Strand V, et al., Arthritis Rheum 2003;48:442-54; Luger D, Dayan M, Zinger H, Liu JP, Mozes E. J Clin Immunol 2004;24:579-90; Mauermann N, Sthoeger Z, Zinger H, Mozes E., Clin Exp lmmunol2004;137:513-20).

Attempts to inhibit B-cell stimulation have focused primarily on receptor-ligand interactions that involve molecules called B lymphocyte stimulator (BLyS) and a proliferationinducing ligand (APRIL). BLyS and APRIL are members of the tumor necrosis factor (TNF) family of cytokines that are critical for B-cell survival and development after exit from the bone marrow. BLyS and APRIL bind to common and distinct receptors. Both molecules bind to transmembrane activator and calciummodulatorand cycolphilin ligand (CAML) interactor (TACI) and B-cell-maturation antigen(BCMA), while BLyS also binds to B-cell-activating factor belonging to the TNF Family -receptor (BAFF-R) and APRIL interacts with proteoglycans.

Mounting evidence in animal models and in humans supports an important role for BLyS and APRIL in the development of autoimmune disease. Transgenic mice that overexpress BLyS display B-cell expansion and polyclonal hypergammaglobulinemia (Gross JA, Johnston J, Mudri S, Enselman R, Dillon S, Madden K, et al., Nature 2000:404:995-9; Mackay F, Woodcock SA, Lawton P, Ambrose C, Baetscher M, Schneider P, et al., J Exp Med 1999;190:1697-710; Khare SD, Sarosi I, Xia XZ, McCabe S, Miner K, Solovyev I, et al., Proc Natl Acad Sci 2000;97:3370-5). Some of these mice develop a lupus-like phenotype consisting of anti-double stranded DNA (dsDNA) antibodies, immunoglobulin deposition in the kidneys, and accelerated development of glomerular disease and levels of BLyS are elevated in lupus-prone NZBINZW F1 (BIW) and MRL-lpr/lpr mice (Stohl W, Xu D, Kim KS, Koss MN, Jorgensen TN, Deocharan B, et al., Arthritis Rheum 2005;52:2080-91). Studies in humans also suggest a role for BLyS and APRIL in systemic autoimmune diseases. Patients with SLE have increased serum levels of BLyS that correlate positively with levels of anti-dsDNA antibodies (Zhang J, Roschke V, Baker K, Wang Z, Alarcon GS, Fessler BJ, et al., JImmunol 2001,166:6-10; Cheema GS, Roschke V, Hilbert DM, Stohl W., Arthritis Rheum 2001;44:1313-19; Stohl W, Metyas S, Tan SM, Cheema GS, Oamar B, Xu D, et al., Arthritis Rheum 2003;48:3475-86). Serum levels of APRIL are elevated in patients with SLE compared with healthy individuals and patients with rheumatoid arthritis (Koyama T, Tsukamoto H, Miyagi Y, Himeji D, Otsuka J, Miyagawa H, et al. Ann Rheum Dis 2005;64:1065-7). BLyS and APRIL have been detected in the synovial fluid of patients with inflammatory arthritis (Tan SM, Xu D, Roschke V, Perry JW, Arkfeld DG, Ehresmann GR, et al., Arthritis Rheum 2003;48:982-92). These compelling observations in mice and humans have led to the developmentof several BLyS antagonists. One of these agents is a recombinant fusion protein comprising the extracellular domain of the TACl receptor joined to a human lgGl Fc domain (atacicept, previously referred to as TACI-Ig). Atacicept blocks B-cell stimulation by both BLyS and APRIL. Several lines of investigation provide support for the expectation that atacicept will have potent effects in vivo. Firstly, transgenic mice that express atacicept have few mature B cells and reduced immunoglobulin concentrations and treatment with atacicept delays the onset and reduces the severity of arthritis in a mouse model of collagen induced arthritis. The production of anti-collagen antibodies is also suppressed (Gross JA, Dillon SR, Mudri S, Johnston J, Littau A, Roque R, et al., supra). Thirdly, treatment of lupus-prone female B/W mice with atacicept delays the development of proteinuria and increases survival (Gross JA, Johnston J, Mudri S, Enselman R, Dillon S, Madden K, et al., supra). Finally, in a direct comparison of the efficacy of murine atacicept and BAFF-R-lg (a BLyS-only inhibitor) in lupus-prone female BIW mice, only atacicept reduced the serum levels of IgM, decreased the frequency of plasma cells in the spleen, and inhibited the IgM response to a T-cell-dependent antigen, suggesting a role for APRIL in these processes (Ramanujam M, Wang X, Huang W, Liu Z, Schiffer L, Tao H, et al., J Clin Invest. 2006; 116:724-34). In light of these encouraging pre-clinical data, the present applicants examined the biologic effects, pharmacokinetics, pharmacodynamics, and safety of atacicept in clinical trials in patients with SLE.

In both exploratory Phase I trials described in Examples 1 and 2 below, atacicept was well tolerated locally and systemically in patients with SLE. Clear signs of atacicept biological activity in this prospective indication, very much in line with its mechanism of action (MoA), have been observed.

According to the current concepts regarding the mechanism of action (MoA) for atacicept and without being bound by theory, the inhibition of BLyS and APRIL results in effects on B-cells, including non-specific and specific antibody secretion, which ultimately affect various SLE-related biomarkers and clinical efficacy markers. As is typical for Phase I studies, the attention in the current analysis is focused on the early stages of the MoA cascade and specifically on the responses starting with early biomarkers of BLyS and APRIL inhibition (such as the atacicept-BLyS complex), and biological effects (such as the Ig levels).

Atacicept displayed multi-phasic, non-linear PK, characterized by a more than dose-proportional increase in free drug exposure and saturated (less than dose-proportional) increase in atacicept-BLyS complex exposure. Such behavior is expected and has been reported in RA patients. It supports the hypothesis that the PK of atacicept are mediated by its ligands. Overall the PK of atacicept, albeit non-linear, were consistent and predictable across the doses, between single and multiple doses. The three PK markers of atacicept behave very similarly in RA and SLE patients which indicates that the type of autoimmune disease is not a major determinant of atacicept PK.

The continuing accumulation of atacicept-BLyS complex with multiple dosing (up to four weekly doses for Cohorts 5 and 6, Study 1), coupled with the minimal accumulation of free atacicept, provide evidence of the presence of considerable initial load of soluble free BLyS and APRIL, both systemically and in the periphery. The elevated baseline BLyS levels measured in these studies (compared to normal subjects from literature) speak in favor of this hypothesis.

It is also very likely that, once the existing pre-dose equilibrium between the soluble ligands and their receptors is disrupted by the administration of atacicept, complex kinetic re-distribution processes between the blood circulation system, the lymphatic system and the periphery compartments are initiated. This re-distribution involves both the drug and its ligands and, given the size of the molecules involved, is likely to take at least several weeks until a new equilibrium is established.

On the other hand, prolonged complex accumulation may imply significant rates of endogenous generation of the free ligands (again both in the blood circulation and in the peripheral tissues). Published data regarding the rate of serum BLyS increase after rituximab administration (Cambridge et al., Arthritis Rheum 2006;54:723-732) seem to provide additional evidence that endogenous BLyS production plays an important role in BLyS inhibition and should be considered. The long time to steady state attainment (beyond one month of weekly dosing) supports those hypotheses.

The saturable kinetics of atacicept was first observed and reported with single atacicept doses applied to healthy volunteers and RA patients in previous Phase I studies and indicates that BLyS (and APRIL) inhibition is saturable, i.e. increasing atacicept exposure beyond the saturation point would bring about diminishing returns in terms of BLyS (and eventually APRIL) binding. This phenomenon should be considered and exploited when selecting therapeutic dosage regimens.

It should be emphasized that the appropriate saturation of BLyS (and APRIL) inhibition needs to be maintained over time, and should be achieved by an appropriate atacicept exposure pattern in time. The latter will require a dynamic balance between the complex and largely uncharacterized processes of endogenous BLyS and APRIL generation and redistribution and the created kinetic profile of atacicept. Such a balance can only be achieved by an appropriate design of the dosing regimen in terms not only of dose levels but also of dosing frequency.

A well-defined relationship between atacicept cumulative dose and Ig antibody response has been established by non-compartmental methods; such a relationship was first detected with single atacicept doses in healthy volunteers and with single and multiple atacicept doses in patients with RA. In the current studies, all three Ig markers monitored showed prompt decreases following the first dose of atacicept. Following four weekly dosing, all three biomarkers of antibody response gradually and consistently decreased toward steady state apparently without reaching it during the dosing period.

The observation that dosing frequency seems to play at least as important a role as dose level in the response of all three biomarkers, first made in the RA study is confirmed with the SLE data after subcutaneous administration (Study 1). In general, the biomarkers behave very similarly at similar dose levels in both the SLE and RA population, underlining the common root in the MoA of both indications based on BLyA (and APRIL) inhibition.

Another interesting fact emerges from the comparison of the PK and biological activity results between the two studies. Although neither of them has been designed to address the subcutaneous availability question, a comparison of the partial and total areas under the concentration - time curves (AUC's) of free and composite atacicept after the similar subcutaneous and intravenous doses (Cohort 3, Study 1 versus Cohort 1 Study 2, and Cohort 4, Study 1 versus Cohort 2, Study 2) permits the derivation of a crude estimate of the "mean" bioavailability. From Tables 2ab and 3ab these estimates are approximately 35-40% for both the free and composite drug - a number that is not outside of the ballpark figures for molecules with large molecular weights (Porter and Charman, J Pharm Sci 2000; 89:297-310).

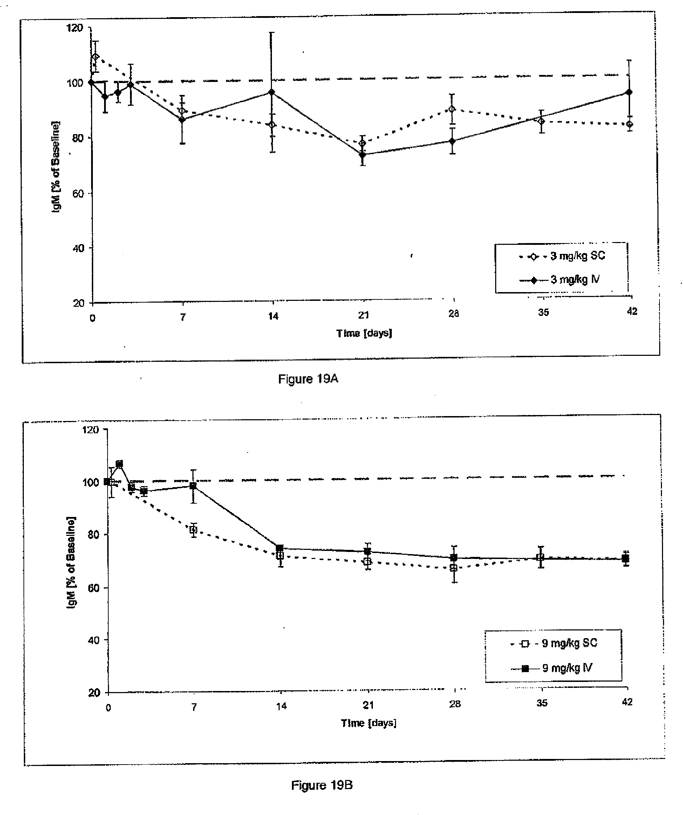

However, the inspection of the biological activity markers reveals that similar doses yield similar biological activity irrespective of the route of administration, as illustrated for IgM in Figure 19. At first take, the observation that 2.5 - 3 fold different systemic exposures to the drug can lead to similar biological effects contradicts the established paradigms; in the atacicept case, however, this phenomenon may be well founded.

The current understanding of the absorption of large protein molecules after subcutaneous administration states that proteins drain from the injection site into both the peripheral lymph and the blood capillaries and the uptake into the lymphatics increases with the molecular size. For drugs with MW commensurate with that of atacicept it can be expected that as much as 70 - 80% of the subcutaneous dose might be expected to go first into the peripheral lymph. However, the blood circulation and the lymphatic systems are so closely inter-wined and connected, that the exchange of mass between the blood and the lymph compartments should be fairly prompt and unimpeded, even for large molecules. The latter is confirmed in the atacicept case by the comparatively (for a MW of 73.4 KDa) rapid equilibration of the intravenous and subcutaneous PK profiles.

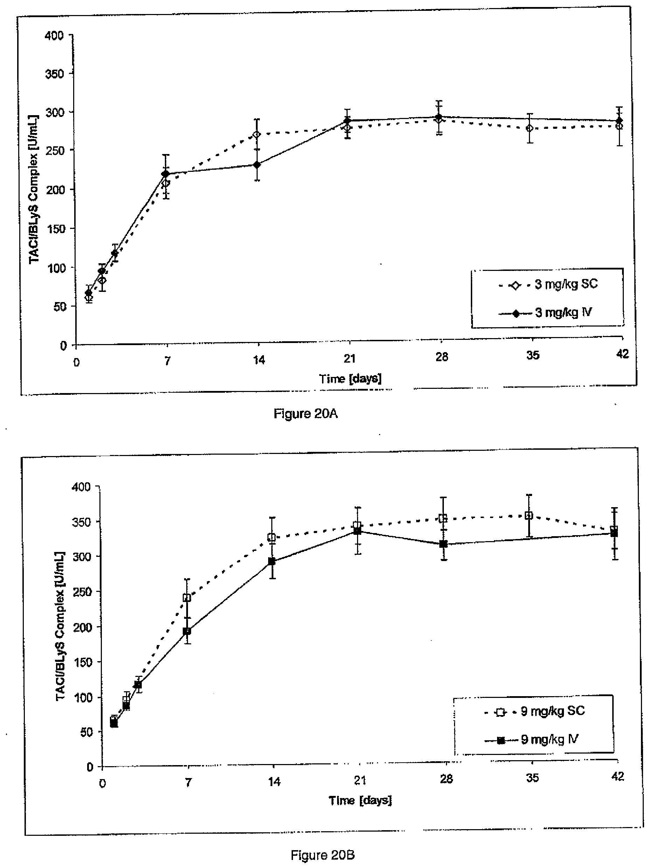

These considerations lead to at least two possible, related, and hence by no means mutually exclusive, explanations of the observed phenomenon. The "kinetic" explanation hypothesizes that even with subcutaneous administration, sufficient amount of drug transfers into the blood circulation to ensure adequate inhibition of BLyS and thus to start the MoA cascade in the central compartment. It is well known that many biological effects are delayed with respect to the underlying drug kinetics. Although in the atacicept case the PD lag is not excessive (as evidenced by the prompt decrease in the Ig markers after the first dose), it seems to be sufficient to render the PK lag caused by the absorption almost irrelevant. This hypothesis is supported by the almost identical atacicept:BLyS complex profiles in the same single dose cohorts of the subcutaneous and intravenous studies (Figure 20), where the similarity is especially noted in the first 7 days post administration.

The "pharmacodynamic" explanation is that both the blood circulation and the lymphatic systems are "sites of action" for BLyS (and APRIL) inhibition and as such represents targets for atacicept. With the intravenous route of administration, the drug is first injected in the blood stream, and from there it distributes to the lymph and other (target and non-target) peripheral tissues. With the subcutaneous route of administration, the drug first drains into the lymphatic compartment and the blood stream in parallel, and from the latter it distributes to the other (target and non-target) peripheral tissues. In both cases penetration of the drug into the both sites of action is immediate and prompt and results into similar biological activity profiles in each of them.

This interesting case supports the hypothesis that the mechanistic application of the paradigm which dictates that assessment of the exposure to subcutaneous administered protein drugs should be done via the systemic or "serum" bioavailability parameter may be inappropriate, or incomplete at best. The rule "the greater the systemic bioavailability - the greater the effect" may have important exceptions in this class of drugs.

The latter has important practical implications related to the development of therapeutic regimen with atacicept. It becomes clear that the intravenous route may be only a vehicle of delivering larger doses of the drug to the patient if such are necessary, given that the magnitude of a subcutaneous dose may be limited by the injection volume and the concentration of the dosing solution.

The good tolerability, marked biological activity of atacicept treatment in line with its MoA, and the other positive trends observed in the two SLE Phase I studies provide the rationale for further research of the drug in patients with SLE. According to the modern drug development science paradigm at each step, newly generated information should be appended to the already existing one, while the drug knowledge base is updated, expanded and improved to be subsequently used for informed design of the next step in a typical 'learn and confirm' cycle. In accordance to that paradigm, we chose to observe and analyze a multitude of exposure (free and composite atacicept), specific binding (atacicept-BLyS complex), biological activity (Igs and immune system cell counts), and certain disease-related markers (anti-dsDNA antibodies) in the two early studies with very complex design (sequential, dose escalating). Analyzing the wealth of data generated in a rigorous way and extracting the information they contain will enable us to define dose ranges and regimens for the further trials that will be needed to characterize the safety profile of atacicept and to enhance the understanding of its MoA, confirm initial indications of clinical efficacy, and define its optimal clinical use.

### Example 1 - Subcutaneous Administration of Atacicept

This phase Ib, double-blind, placebo-controlled, dose-escalating trial comprised six cohorts (n=8 each, except for cohort 5, n='7) of patients treated with atacicept or placebo in a 3:1 ratio. Cohorts 1-4 received a single subcutaneous dose of placebo, or 0.3, 1, 3, or 9 mg/kg of atacicept. Cohorts 5 and 6 received four weekly doses of placebo, or 1 or 3 mg/kg of atacicept (see Table 1). Patients were followed for 6 (cohorts 1-4) or 9 (cohorts 5 and 6) weeks. Outcome measures included: (i) systemic and local tolerability of atacicept; (ii) frequency of adverse events (AEs); (iii) pharmacokinetics and pharmacodynamics of atacicept, including effects on lymphocyte subpopulations and Ig levels; and (iv) measures of SLE disease activity.

Patients with mild-to-moderate SLE were enrolled. Biologic activity of atacicept was demonstrated by dose-dependent reductions in immunoglobulin levels and in mature and total B cells. This effect was most pronounced in the repeat-dose cohorts and was sustained throughout the follow-up period. There were no changes in the numbers of T cells, natural killer cells, or monocytes. Mild injection-site reactions occurred more frequently among the atacicept than the placebo group. There were no differences in the frequency or type of adverse events, and no severe or serious adverse events in patients treated with atacicept.

Pharmacokinetics were assessed by measuring serum levels of free atacicept (Table 2a), atacicept/BLyS complex (Table 3a), and composite atacicept (defined as free atacicept + atacicept-BLyS complex, Table 4a). Serum levels of each of these were quantified using an enzyme-linked immunosorbant assay. Serum was incubated with a biotin-conjugated mouse mAb specific for atacicept (free or total atacicept detection) (ZymoGenetics, Inc., Seattle, WA) or biotin-conjugated goat polyclonal antibodies specific for either BLyS or atacicept (atacicept/BLyS complex detection) (R & D Systems, Minneapolis, MN), immobilized on a streptavidin-coated microplate (Adaltis, Montreal, Quebec). The antibodies were incubated together with patient samples, standard, or control samples diluted 1:10 for 1 hour. After washing, an atacicept-specific mouse mAb conjugated to horseradish peroxidase (HRP) (to measure free atacicept or atacicept-BLyS complex) or in the case of composite ELISA, mAbs against atacicept and BLyS (ZymoGenetics, Inc.) are added and incubated at room temperature for 1 hour. In all three assays, atacicept serum levels were detected and quantified using standard chemiluminescence methods, i.e., after washing tetramethylbenzidine (TMB) was added as HRP substrate (Sigma-Aldrich, St. Louis, MO). The reaction was halted after 20 minutes using 0.5 M sulfuric acid and the absorbance recorded at 450 nm. The analyte concentration of a patient sample was recalculated using the standard curve, applying a polynomial second order-fitting algorithm. All samples were measured in triplicate. Assay performance criteria of a precision of <15% coefficient of variation (CV) for standard samples and <20% for patient samples were accepted. The lower limits of quantification (LLOQ) of the assays were 15.6 ng/mL for free atacicept, 5 U/mL for atacicept-BLyS complex (1 U/mL corresponding to 1.82 ng/mL atacicept-0.44 ng/mL BLyS in a 3:1 molar ratio), and 25 ng/mL for the composite analytes. The mean spiking recoveries performed to test the accuracy for low, medium and high analyte concentrations in RA patient samples corresponded to recovery rates of 82.5-97.0%, 93.9%, and 102.0-125.8% in the three assays, respectively. Serum PK markers were sampled as follows: (i) for the single-dose Cohorts 1-4 - at baseline and at 4, 8, 12 hours on the day of administration and thereafter on study Days 2, 3, 4, 8, 15, 22, 29, and 43; (ii) for the multiple-dose arms in Cohorts 5 and 6 - at baseline and thereafter on study Days 8, 15, 22, 29, 36, 43, 64. In all cohorts PK samples on dosing days were specified nominally as troughs.

Unbound BLyS concentrations were measured in serum at baseline. BLyS was measured by ELISA. Biotinylated mAbs specific for BLyS were incubated together with patient samples, standard or control samples (diluted 1:10) for 1 hour in streptavidin pre-coated microplates. After washing, anti-BLyS, HRP-conjugated mouse mAbs were incubated at room temperature for 1 hour. After washing, TMB was added as HRP substrate. The reaction was stopped after 20 minutes using 0.5 M sulfuric acid and the absorbance recorded at 450 nm. The analyte concentration of a patient sample was recalculated using the standard curve applying a polynomial second order-fitting algorithm. All samples were measured in triplicate. Assay performance criteria of a precision of <20% CV was an accepted measurement in patient samples. The LLOQ was 1.56 ng/mL BLyS in serum. The mean spiking recoveries for low, medium and high concentration of analytes in RA patient samples corresponded to recovery rates of 101-113%.

Pharmacodynamics were assessed by measuring serum levels of immunoglobulins (IgG, IgM, IgA), complement-3 (C3), and anti-nuclear antibodies (ANA), and by performing flow cytometry analysis of lymphocyte subsets. Immunoglobulins and C3 were measured using standard methods. ANA were measured using the Athena Multianalyte ANA test system (Zeus Scientific Inc, Raritan, NJ, USA). IgG, IgM, and IgA were assessed in the blood as markers of biological activity. The biomarkers were measured at baseline and at 8 hours on the day of administration (Cohorts 1-4 only), and thereafter on study Days 8, 15, 22, 29, 36, 43, and 64.

A panel of peripheral blood mononuclear cell types (B- and T-cell subsets, natural killer [NK] cells and monocytes) was assessed in antibody-stained peripheral blood samples, using four-color flow cytometry. The analysis included: total T cells (CD45+, CD3+), T-helper cells (CD45+, CD3+, CB4+, CD8-), T-cytotoxic/suppressor cells (CD45+, CD3+,CD4-, CD8+), total B cells (CDI 9+), mature B cells (CDI 9+, IgD+, CD27-), monocytes (CD45+, CD3-, CD14+, CD56-), and NK cells (CD45+, CD3-, CD14-, CD56+). A contract research organization (Esoterix, Groningen, The Netherlands) performed blood sample processing, antibody staining, and acquisition, analysis and quality control of data. We performed further analysis and quality control on B-cell subsets. For B-cell subsets, the analysis gate was enlarged to include small and large lymphocytes, with the latter being similar in size to monocytes.

Medical history was collected at inclusion and a physical examination was conducted on a weekly basis. Hematologic, and serum chemistry profiles were performed on a weekly basis and evaluated using the National Cancer Institute's Common Toxicity Criteria. Blood samples for pharmacokinetic evaluations were collected on a weekly basis for repeat-dose cohorts and on Day 1 at Hours 4 and 8, Days 2,3,4 and 8 and on a weekly basis thereafter for the single-dose cohorts. Blood samples for pharmacodynamic evaluations were drawn on a weekly basis in the repeat-dose cohorts and on Days 2, 3, 8, and then on a weekly basis in the single-dose cohorts.

Electrocardiogram after D4 was performed on a bi-weekly basis in the single-dose cohorts and on a weekly basis in patients receiving repeated doses of the study drug.

Although the study was not powered to determine the impact of treatment on disease activity, the following disease activity measurements were obtained to provide preliminary efficacy data. SELENA SLEDAI scores were determined at baseline and at Days 29 and 43 (cohorts 1-4) and at Days 22 and 64 (cohorts 5 and 6). Anti-dsDNA antibody and C3 levels were measured at baseline and at Days 15, 29, and 43 (cohortsl-4), and at Days 15, 22, 29,43, and 64 (cohorts 5 and 6).

The data analysis methods included subjecting concentration-time profiles were subjected to non-compartmental analysis (NCA; WinNonLin software, version 5.0.1). All measurements below the LLOQ were ignored for the NCA. Biomarker (IgM, IgG, or IgA) data were converted into 'change from baseline' format and then the individual biomarker-time profiles were also subjected to NCA. The resulting NCA-derived measures for exposure (PK) and response were subsequently analyzed together to explore the existing exposure-response relationships.

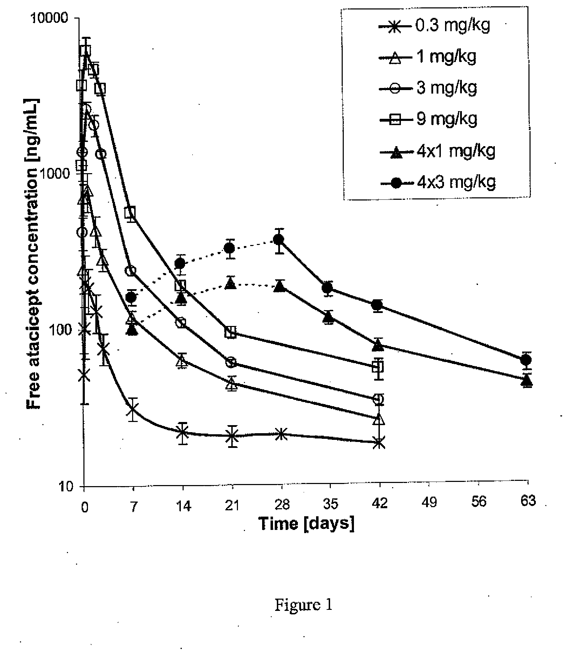

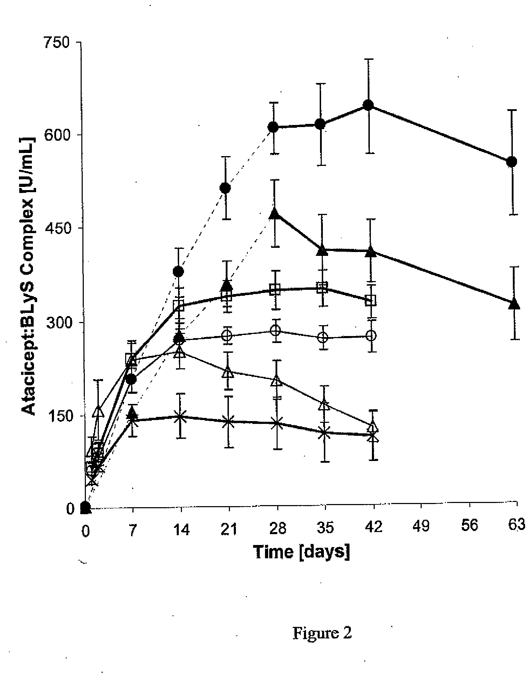

Evidence of non-linear pharmacokinetics, consistent with saturable binding pharmacokinetics of ligand-receptor interactions, was demonstrated (Figures 1 and 2). Free and composite atacicept concentration-time profiles displayed multiphasic pharmacokinetics with fairly rapid absorption, Tmax approximately 24 hours after the first dose, and an initial distribution phase lasting 7-14 days. Low accumulation of free atacicept was observed in the repeat-dose cohort; the accumulation of composite atacicept was marginally higher and the atacicept-BlyS complex was found to accumulate throughout the dosing period.

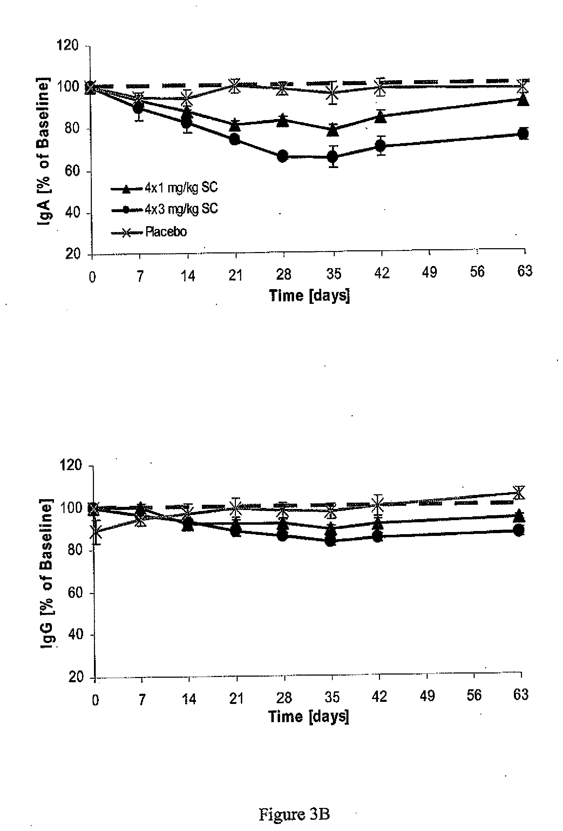

Treatment with atacicept was associated with an initial, transient increase in mature and total B cells followed by a sustained, dose-related reduction (Figure 3B). In the single dose 3 mg/kg and 9 mg/kg groups and in the repeat-dose groups a reduction from baseline of approximately 35% in mature B cells was seen at Day 29. In the single-dose groups, this reduction was sustained through to Day 43; in the repeat-dose groups, a reduction of approximately 60% was seen at Day 43 and was sustained at 45-60% through to the last assessment at Day 64. The patterns observed for total B cells were similar to those for mature B cells. In the 3 mg/kg single-dose group, a reduction from baseline of approximately 30% in total B cells was seen at Day 29, which was sustained through to Day 43. In the repeat-dose groups, reductions of approximately 40-50% were seen at Day 43 and were sustained at 35-60% through to the last assessment at Day 64 (Figure 3B). There were no significant changes in the number of total, helper, or cytotoxic T cells, NK cells, or monocytes.

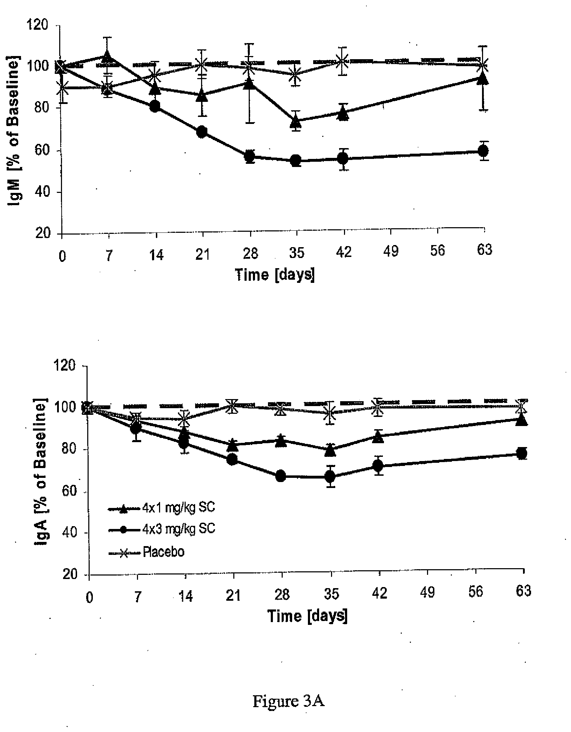

Dose-dependent reductions in immunoglobulin levels were observed in atacicept treated patients (Figure 3A and 3B, see also Table 5a). This effect was most notable in the repeat-dose groups. IgM levels showed the greatest declines with treatment, reaching nearly 50% at Day 43 in the 3 mg/kg repeat-dose group. IgA levels decreased by approximately 33% in the 3 mg/kg repeat-dose group at Day 29, and IgG levels decreased by approximately 16% in the 3 mg/kg repeat-dose group at Day 36. Nadirs occurred between Days 15 and 29 in the single-dose cohorts and between Days 29 and 43 in the repeat-dose cohorts.

Thereafter, values began to return towards baseline. Last observed values were approximately 5-30% below baseline in the single-dose cohorts (with the exception of the 0.3 mg/kg group where IgM values were above baseline) and 8-65% below baseline in the repeat-dose cohorts.

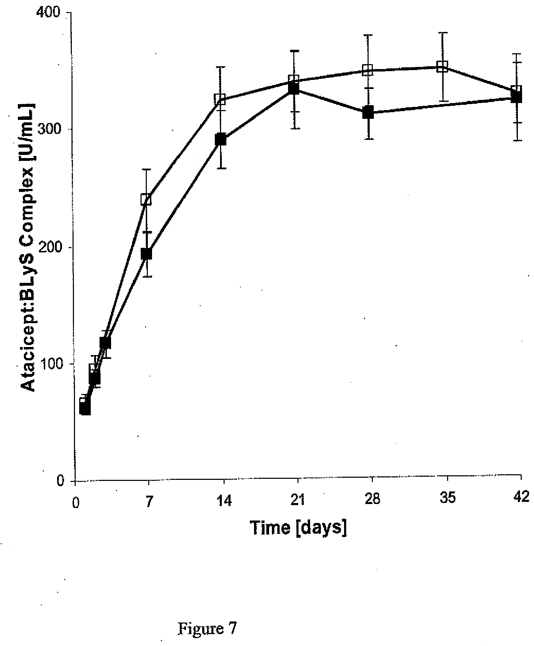

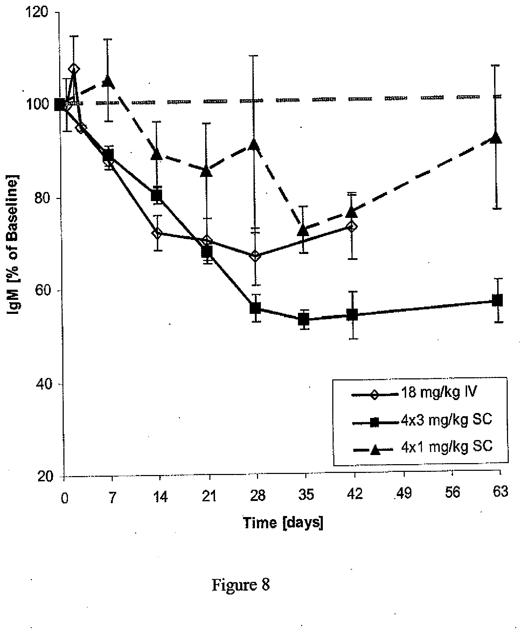

These results indicate that more frequent administration of smaller doses of atacicept yield better biological activity than less frequent dosing with higher doses (Figure 7 and Figure 8).

### Example 2 - Intravenous Administration of Atacicept

This phase Ib, double-blind, placebo-controlled, dose-escalating trial comprised four cohorts (n=6 each) of patients treated intravenously with atacicept or placebo in a 3:1 ratio. Cohorts 1-3 received a single dose of placebo, 3, 9, or 18 mg/kg of atacicept. Cohort 4 received two doses of placebo or 9 mg/kg of atacicept, the second dose occurring at three weeks after the initial dose (see Table 1). Outcome measures included: (i) systemic and local tolerability of intravenous atacicept; (ii) frequency of adverse events (AEs); (iii) pharmacokinetics and pharmacodynamics of intravenous atacicept, including effects on lymphocyte subpopulations and Ig levels; and (iv) measures of SLE disease activity. Subjects were evaluated over a 6-week (cohorts 1-3) or 9-week (cohort4) period; subjects from cohorts 3 and 4 returned at study days 84 and 120 for PK and biomarker sampling. Serum PK markers were sampled as follows: (i) for the single-dose Cohorts 1-3 - at baseline and at 0.25, 0.5, 4 hours on the day of administration and thereafter on study Days 2, 3, 4, 8, 15, 22, 29, and 43; (ii) for the multiple-dose Cohort 4 - at baseline and at 0.25, 0.5, 4 hours on the day of the first administration and thereafter on study Days 8, 22, 22 (before the second dose and 0,25 and 0.5 h after the second dose), 29, 36, 43, 64. Cohorts 3 and 4 have PK measurements on study Days 85 and 120. In all cohorts PK samples on dosing days were specified nominally as troughs. Unbound BLyS concentrations were measured in serum at baseline. IgG, IgM, and IgA were assessed in the blood as markers of biological activity. The biomarkers were measured at baseline, and thereafter on study Days 2, 3, 4, 8, 15 (Cohorts 1-3 only), 22, 29, 36, 43, and 64 (Cohort 4 only). Cohorts 3 and 4 have Ig measurements on study Days 85 and 120 as well.

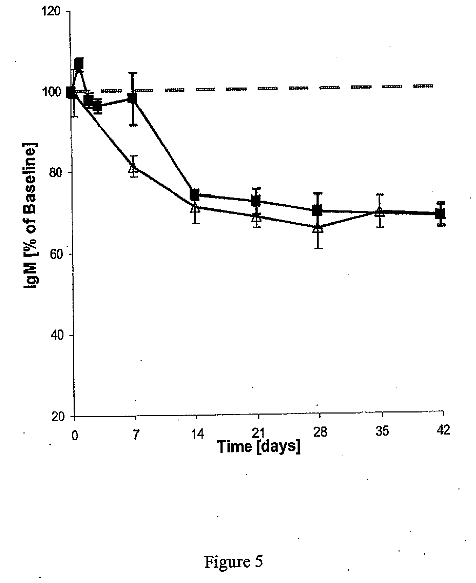

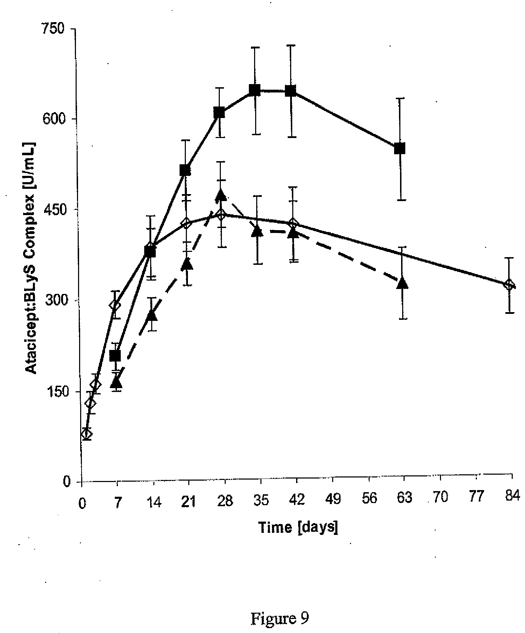

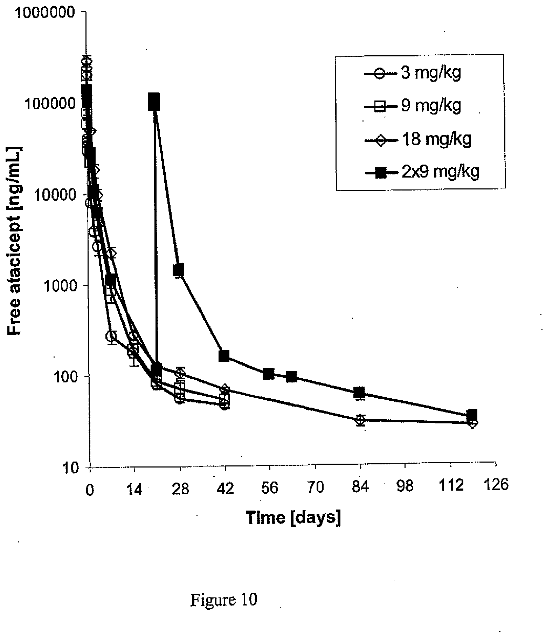

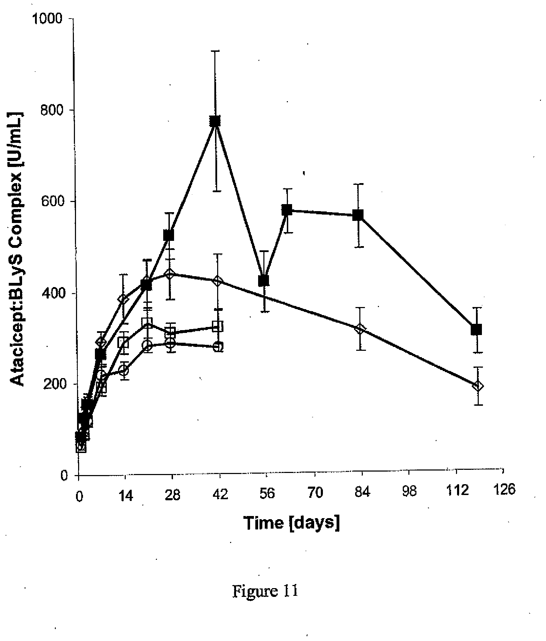

As with Example 1, patients with mild-to-moderate SLE were enrolled and pharmacokinetics were assessed by measuring serum levels of free atacicept (Table 2b), atacicept/BLyS complex (Table 3b), and composite atacicept (defined as free atacicept + atacicept-BLyS complex, Table 4b)). Biologic activity of atacicept was demonstrated by dose-dependent reductions in immunoglobulin levels and in mature and total B cells (see Figure 11, see also Figure 5b). This effect was most pronounced in the repeat-dose cohort and was sustained throughout the follow-up period. There were no changes in the numbers of T cells, natural killer cells, or monocytes. Mild administration site reactions occurred more frequently among the atacicept than the placebo group. There were no differences in the frequency or type of adverse events, and no severe or serious adverse events in patients treated with atacicept. Comparison between the subcutaneous administration (see Example 1) and intravenous administration routes revealed very similar pharmacokinetics (nonlinear PK mediated by ligands) and a similar PK which was predictable and consistent with single and multiple doses (Figures 9 and 10).

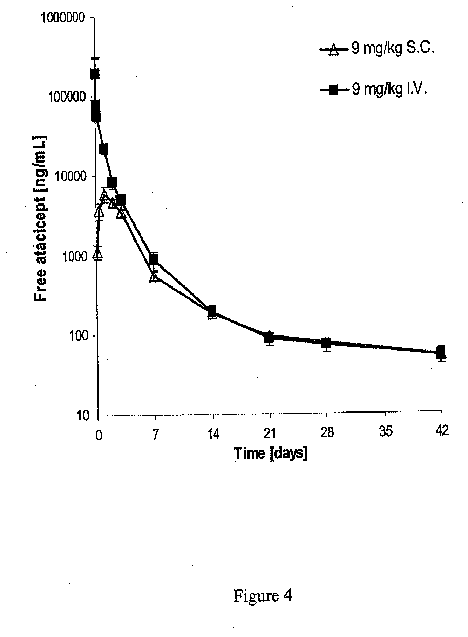

Although there did not appear to be an advantage to intravenous administration (despite higher bioavailability with this route of administration, see Figure 4) these results also support the conclusion that more frequent administration of smaller doses of atacicept yield better biological activity than less frequent dosing with higher doses (Figure 7 and Figure 8). These results also indicate that despite the lower bioavailability of the drug using subcutaneous administration, the binding profile of the two methods are very comparable (see Figure 7).

**Table 1. Dosing arms in the Phase I SLE studies with atacicept.**

| Study 1 - subcutaneous atacicept | | | |
|---|---|---|---|
| Cohort | Dose | Administration | Number of patients |
| 1 | 1x0.3 mg/kg | Single dose | 6 active, 2 placebo |
| 2 | 1x1 mg/kg | Single dose | 6 active, 2 placebo |
| 3 | 1x3 mg/kg | Single dose | 6 active, 2 placebo |
| 4 | 1x9 mg/kg | Single dose | 6 active, 2 placebo |
| 5 | 4x1 mg/kg | 4 weekly doses (QW) | 6 active, 2 placebo |
| 6 | 4x3 mg/kg | 4 weekly doses (QW) | 6 active, 2 placebo |

| Study 2 - intravenous atacicept | | | |
|---|---|---|---|
| Cohort | Dose | Administration | Number of patients |
| 1 | 1x3 mg/kg | Single dose | 5 active, 1 placebo |
| 2 | 1x9 mg/kg | Single dose | 5 active, 1 placebo |
| 3 | 1x18 mg/kg | Single dose | 5 active, 1 placebo |
| 4 | 2x9 mg/kg | 2 doses 3 weeks apart | 5 active, 1 placebo |

| | | | |
|---|---|---|---|
| QW, every week | | | |

**Table 2a. Non-compartmental analysis-derived pharmacokinetic parameters for free atacicept, Study 1.**

| Estimated after the first dose | | | | | |
|---|---|---|---|---|---|
| | T_{½} (hours) | Tₘₐₓ (hours) | Cₘₐₓ (ng/mL) | AUC_{INF} (mg·h/L) | AUC₃₃₆ (mg·h/L) |
| Cohort 1, 0.3 mg/kg | | | | | |
| Mean (SD) | 401 (477) | 28 (9.80) | 185 (145) | 30.4 (29.4) | 17.6(10.6) |
| Median | 204 | 24 | 136 | 19.1 | 14.1 |
| Cohort 2, 1 mg/kg | | | | | |
| Mean (SD) | 452(219) | 18.7 (8.26) | 821 (525) | 108 (38.4) | 69.3 (29.1) |
| Median | 433 | 24 | 666 | 120 | 67.9 |
| Cohort 3, 3 mg/kg | | | | | |
| Mean (SD) | 651 (218) | 28 (9.80) | 2600 (745) | 287 (55.6) | 218(47.2) |
| Median | 572 | 24 | 2830 | 293 | 239 |
| Cohort 4, 9 mg/kg | | | | | |
| Mean (SD) | 642(218) | 32(12.4) | 6190 (3200) | 634(141) | 520(157) |
| Median | 653 | 24 | 5370 | 608 | 474 |

| Estimated after the last dose | | | | | |
|---|---|---|---|---|---|
| Cohort 5, 4x1 mg/kg | | | | | |
| Mean (SD) | 690 (230) | N.E. | N.E. | 147 (24.1) | 56.9 (9.80) |
| Median | 729 | N.E. | N.E. | 149 | 59.3 |
| Cohort 6, 4x3 mg/kg | | | | | |
| Mean (SD) | 472 (157) | N.E. | N.E. | 209 (33.7) | 96.5 (34.5) |
| Median | 492 | N.E. | N.E. | 202 | 85.8 |

| | | | | | |
|---|---|---|---|---|---|
| AUC₃₃₆, area under the concentration-time curve from time 0 hours to time 336 hours; AUC_{INF}, AUC from time 0 hours to infinity; Cₘₐₓ, maximum concentration; SD, standard deviation; T_{½}, terminal half-life; Tₘₐₓ, time of the maximum concentration. Last dose administered at 504 h. N=6 SLE patients per cohort. N.E. - Not estimated due to sampling scheme after the last dose. | | | | | |

**Table 2b. Non-compartmental analysis-derived pharmacokinetic parameters for free atacicept, Study 2.**

| Estimated after the first dose | | | | | |
|---|---|---|---|---|---|
| | T_{½} (hours) | Tₘₐₓ (hours) | Cₘₐₓ (ng/mL) | AUC_{INF} (mg·h/L) | AUC₃₃₆ (mg·h/L) |
| Cohort 1, 3 mg/kg | | | | | |
| Mean (SD) | 743 (216) | 0.500 (0) | 39.7 (5.49) | 912(189) | 815(170) |
| Median | 642 | 0.500 | 38.6 | 953 | 848 |
| Cohort 2, 9 mg/kg | | | | | |
| Mean (SD) | 722 (146) | 0.350 (0.137) | 198 (248) | 2040 (613) | 1930 (565) |
| Median | 765 | 0.250 | 91.7 | 1770 | 1680 |
| Cohort 3, 18 mg/kg | | | | | |
| Mean (SD) | 796 (188) | 0.400 (0.137) | 289 (91.2) | 5010(743) | 4840 (713) |
| Median | 702 | 0.500 | 257 | 4880 | 4730 |
| Cohort 4, 2x9 mg/kg three weeks apart | | | | | |
| Mean (SD) | N.E. | 1.15 (1.60) | 140 (23.7) | N.E. | 275 (220) |
| Median | N.E. | 0.500 | 148 | N.E. | 2620 |
| Estimated after the last dose | | | | | |
| Cohort 4, 2x9 mg/kg three weeks apart | | | | | |
| Mean (SD) | 748 (92.2) | 504.25 (0) | 109 (22.6) | 4320(1020) | 4110(971) |
| Median | 710 | 504.25 | 119 | 4700 | 4520 |

| | | | | | |
|---|---|---|---|---|---|
| AUC₃₃₆, area under the concentration-time curve from time 0 hours to time 336 hours; AUC_{INF}, AUC from time 0 hours to infinity; Cₘₐₓ, maximum concentration; SD, standard deviation; T_{½}, terminal half-life; Tₘₐₓ, time of the maximum concentration. Last dose administered at 504 h. N=6 SLE patients per cohort. N.E. - Not estimated due to sampling scheme after the first dose. | | | | | |

**Table 3a. Non-compartmental analysis-derived pharmacokinetic parameters for composite atacicept, Study 1.**

| Estimated after the first dose | | | | | |
|---|---|---|---|---|---|
| | T_{½} (hours) | Tₘₐₓ (hours) | Cₘₐₓ (ng/mL) | AUC_{INF} (mg·h/L) | AUC₃₃₆ (mg·h/L) |
| Cohort 1, 0.3 mg/kg | | | | | |
| Mean (SD) | 3710(5450) | 30(14.7) | 436 (300) | 1360 (2120) | 75.6 (36.5) |
| Median | 1270 | 24 | 324 | 319 | 61.0 |
| Cohort 2, 1 mg/kg | | | | | |
| Mean (SD) | 807 (515) | 16(8.80) | 1160 (597) | 610(370) | 168 (47.1) |
| Median | 543 | 16 | 963 | 441 | 169 |
| Cohort 3, 3 mg/kg | | | | | |
| Mean (SD) | 3040 (2770) | 32(12.4) | 3140 (935) | 2470(1620) | 400 (69.3) |
| Median | 2580 | 24 | 3130 | 2210 | 411 |
| Cohort 4, 9 mg/kg | | | | | |
| Mean (SD) | 878 (256) | 32(12.4) | 8890 (4860) | 1770 (378) | 865 (199) |
| Median | 795 | 24 | 7150 | 1780 | 807 |

| Estimated after the last dose | | | | | |
|---|---|---|---|---|---|
| Cohort 5, 4x1 mg/kg | | | | | |
| Mean (SD) | 1410 (572) | N.E. | N.E. | 1570 (834) | 237 (50.4) |
| Median | 1610 | N.E. | N.E. | 1490 | 221 |
| Cohort 6, 4x3 mg/kg | | | | | |
| Mean (SD) | 1500 (545) | N.E. | N.E. | 2530 (1340) | 386 (83.4) |
| Median | 1520 | N.E. | N.E. | 2070 | 389 |

| | | | | | |
|---|---|---|---|---|---|
| AUC₃₃₆, area under the concentration-time curve from time 0 hours to time 336 hours; AUC_{INF}, AUC from time 0 hours to infinity; Cₘₐₓ, maximum concentration; SD, standard deviation; T_{½}, terminal half-life; Tₘₐₓ, time of the maximum concentration. Last dose administered at 504 h. N=6 SLE patients per cohort; N.E. - Not estimated due to sampling scheme after the last dose. | | | | | |

**Table 3b. Non-compartmental analysis-derived pharmacokinetic parameters for composite atacicept, Study 2.**

| Estimated after the first dose | | | | | |
|---|---|---|---|---|---|
| | T_{½} (hours) | Tₘₐₓ (hours) | Cₘₐₓ (ng/mL) | AUC_{INF} (mg·h/L) | AUC₃₃₆ (mg·h/L) |
| Cohort 1, 3 mg/kg | | | | | |
| Mean (SD) | 2550 (1220) | 0.350 (0.137) | 57.3 (12.0) | 2870 (531) | 1180 (184) |
| Median | 2050 | 0.250 | 55.9 | 2650 | 1200 |
| Cohort 2, 9 mg/kg | | | | | |
| Mean (SD) | 1560 (842) | 0.350 (0.137) | 348 (362) | 4590 (520) | 3210 (650) |
| Median | 1600 | 0.250 | 201 | 4430 | 2880 |
| Cohort 3, 18 mg/kg | | | | | |
| Mean (SD) | 1080 (147) | 0.250 (0) | 411 (77.3) | 7470 (1380) | 5990 (863) |
| Median | 1100 | 0.250 | 446 | 7710 | 6240 |
| Cohort 4, 2x9 mg/kg three weeks apart | | | | | |
| Mean (SD) | N.E. | 1.05 (1.65) | 361 (334) | N.E. | 4720 (2400) |
| Median | N.E. | 0.250 | 251 | N.E. | 4320 |

| Estimated after the last dose | | | | | |
|---|---|---|---|---|---|
| Cohort 4, 2x9 mg/kg three weeks apart | | | | | |
| Mean (SD) | 148 (750) | 504.25 (0) | 240 (69.4) | 8490 (2010) | 6390 (1200) |
| Median | 1140 | 504.25 | 272 | 8130 | 6390 |

| | | | | | |
|---|---|---|---|---|---|
| AUC₃₃₆, area under the concentration-time curve from time 0 hours to time 336 hours; AUC_{INF}, AUC from time 0 hours to infinity; Cₘₐₓ, maximum concentration; SD, standard deviation; T_{½}, terminal half-life; Tₘₐₓ, time of the maximum concentration. Last dose administered at 504 h. N=6 SLE patients per cohort. N.E. - Not estimated due to sampling scheme after the first dose. | | | | | |

**Table 4a. Non-compartmental analysis-derived pharmacokinetic parameters for BLyS-atacicept complex, Study 1.**

| Estimated after the first dose | | | | | |
|---|---|---|---|---|---|
| | *T_{½} (hours) | Tₘₐₓ (hours) | Cₘₐₓ [kU/mL] | *AUC_{INF} (kU·h/L) | AUC₃₃₆ (kU·h/L) |
| Cohort 1, 0.3 mg/kg | | | | | |
| Mean (SD) | 1240 (656) | 364 (126) | 161 (91.9) | 382 (390) | 38.5 (17.8) |
| Median | 1230 | 336 | 134 | 259 | 33.7 |
| Cohort 2, 1 mg/kg | | | | | |
| Mean (SD) | 821 (704) | 260 (124) | 269 (89.9) | 362 (196) | 68.7 (24.1) |
| Median | 512 | 336 | 286 | 319 | 73.0 |
| Cohort 3, 3 mg/kg | | | | | |
| Mean (SD) | 1250 (517) | 728 (330) | 316(30.4) | 727 (323) | 59.5 (13.3) |
| Median | 1420 | 840 | 315 | 735 | 55.9 |
| Cohort 4, 9 mg/kg | | | | | |
| Mean (SD) | 6960 (6550) | 700 (223) | 369 (66.2) | 3680 (2890) | 69.9 (16.7) |
| Median | 6220 | 672 | 388 | 4330 | 72.7 |

| Estimated after the last dose | | | | | |
|---|---|---|---|---|---|
| Cohort 5, 4x1 mg/kg | | | | | |
| Mean (SD) | 2510 (2330) | 756 (92.0) | 460 (122) | 1700 (1330) | 139 (38.1) |
| Median | 1970 | 756 | 443 | 1260 | 134 |
| Cohort 6, 4x3 mg/kg | | | | | |
| Mean (SD) | 7680 (13200) | 840 (184) | 668 (159) | 8050 (13700) | 201 (40.6) |
| Median | 2220 | 840 | 660 | 2450 | 199 |

| | | | | | |
|---|---|---|---|---|---|
| AUC₃₃₆, area under the concentration-time curve from time 0 hours to time 336 hours; AUC_{INF}, AUC from time 0 hours to infinity; Cₘₐₓ, maximum concentration; SD, standard deviation; T_{½}, terminal half-life; Tₘₐₓ, time of the maximum concentration. Last dose administered at 504 h. N=6 SLE patients per cohort. * T_{1/2} and AUC_{INF} are not reliable estimates for this variable due to the terminal shape of the profiles. | | | | | |

**Table 4b. Non-compartmental analysis-derived pharmacokinetic parameters for BLyS-atacicept complex, Study 2.**

| Estimated after the first dose | | | | | |
|---|---|---|---|---|---|
| | *T_{½} (hours) | Tₘₐₓ (hours) | Cₘₐₓ (kU/mL) | *AUC_{INF} (kU·h/L) | AUC₃₃₆ (kU·h/L) |
| Cohort 1, 3 mg/kg | | | | | |
| Mean (SD) | 8650 (159000) | 672 (206) | 0.297 (0.0412) | 35100 (63800 | 59.6 (10.2) |
| Median | 9800 | 672 | 0.300 | 4240 | 63.1 |
| Cohort 2, 9 mg/kg | | | | | |
| Mean (SD) | 3790 (2320) | 605 (255) | 0.352 (0.0763) | 2040 (1230) | 61.0(11.4) |
| Median | 3770 | 504 | 0.342 | 2480 | 58.8 |
| Cohort 3, 18 mg/kg | | | | | |
| Mean (SD) | 1800 (616) | 672 (206) | 0.454 (0.118) | 1460 (674) | 86.3 (20.0) |
| Median | 1570 | 672 | 0.452 | 1210 | 84.3 |
| Cohort 4, 2x9 mg/kg three weeks apart | | | | | |
| Mean (SD) | N.E. | 504 (0) | 0.414 (0.121) | N.E. | 78.8 (17.8) |
| Median | N.E. | 504 | 0.397 | N.E. | 78.4 |

| Estimated after the last dose | | | | | |
|---|---|---|---|---|---|
| Cohort 4, 2x9 mg/kg three weeks apart | | | | | |
| Mean (SD) | 265 (3270) | 1008 (0) | 0.707 (0.343) | 2790 (2550) | 186 (38.0) |
| Median | 1140 | 1008 | 0.685 | 1740 | 195 |

| | | | | | |
|---|---|---|---|---|---|
| AUC₃₃₆, area under the concentration-time curve from time 0 hours to time 336 hours; AUC_{INF}, AUC from time 0 hours to infinity; Cₘₐₓ, maximum concentration; SD, standard deviation; T_{½}, terminal half-life; Tₘₐₓ, time of the maximum concentration. Last dose administered at 504 h. N=6 SLE patients per cohort. N.E. - not estimated * T_{1/2} and AUC_{INF} are not reliable estimates for this variable due to the terminal shape of the profiles. | | | | | |

**Table 5a. Non-compartmental analysis results for immunoglobulin (Ig)M, IgA, and IgG biomarkers - Study 1.**

| Cohort* | IgM | | IgA | | IgG | |
|---|---|---|---|---|---|---|
| | Tₘₐₓ (days) | Max change (% of baseline) | Tₘₐₓ (days) | Max change (% of baseline) | Tₘₐₓ (days) | Max change (% of baseline) |
| Cohort 1, 0.3 mg/kg s.c. | | | | | | |
| Mean (SD) | 14.1 (17) | 2.82 (23.4) | n.d. | n.d. | 11.8 (15.7) | -0.377 (17.8) |
| Median | 7 | 10.2 | n.d. | n.d. | 7.00 | 4.07 |
| Cohort 2, 1 mg/kg s.c. | | | | | | |
| Mean (SD) | 18.7 (13.0) | 19.4 (8.93) | n.d. | n.d. | 25.7 (14.5) | 11.1 (5.77) |
| Median | 17.5 | 16.5 | n.d. | n.d. | 24.5 | 12.1 |
| Cohort 3, 3 mg/kg s.c. | | | | | | |
| Mean (SD) | 23.3 (5.70) | 24.8 (3.81) | n.d. | n.d. | 29.2 (8.20) | 10.5 (1.25) |
| Median | 21.0 | 25.2 | n.d. | n.d. | 28.0 | 10.9 |
| Cohort 4, 9 mg/kg s.c. | | | | | | |
| Mean (SD) | 29.2 (8.20) | 37.9 (8.57) | n.d. | n.d. | 18.7 (7.20) | 14.7 (5.95) |
| Median | 28.0 | 37.2 | n.d. | n.d. | 21.0 | 15.9 |
| Cohort 5, 4x1 mg/kg s.c. QW | | | | | | |
| Mean (SD) | 31.0(11.3) | 29.2 (10.0) | 27.0 (10.2) | 23.1 (10.1) | 25.0 (11.3) | 11.5 (5.84) |
| Median | 35.0 | 31.0 | 28.0 | 20.7 | 28.0 | 14.4 |
| Cohort 6 4x3 mg/kg s.c. QW | | | | | | |
| Mean (SD) | 42.0 (11.7) | 50.4 (7.25) | 36.2 (2.90) | 34.9 (5.85) | 33.8 (6.90) | 17.5 (4.22) |
| Median | 42.0 | 49.8 | 35.0 | 32.9 | 35.0 | 17.8 |
| Placebo^{†} | | | | | | |
| Mean (SD) | 13.5 (12.4) | 13.7 (15.3) | 14.0 (9.90) | 9.14 (7.06) | 11.0 (11.3) | 11.2 (13.5) |
| Median | 7.00 | 10.9 | 10.5 | 10.6 | 14.0 | 9.84 |

| | | | | | | |
|---|---|---|---|---|---|---|
| *Active-dose patients for Cohorts 1-6 (n=6) †Placebo cohort, all placebo patients pooled together (n=12). Tₘₐₓ, time of the maximum depletion of Ig. n.d. - no data. | | | | | | |

**Table 5b. Non-compartmental analysis results for immunoglobulin (Ig)M, IgA, and IgG biomarkers - Study 2.**

| Cohort* | IgM | | IgA | | IgG | |
|---|---|---|---|---|---|---|
| | Tₘₐₓ (days) | Max change (% of baseline) | Tₘₐₓ (days) | Max change (% of baseline) | Tₘₐₓ (days) | Max change (% of baseline) |
| Cohort 1, 3 mg/kg i.v. | | | | | | |
| Mean (SD) | 23.8 (12.7) | 34.2 (11.2) | 18.2 (14.5) | 19.0 (10.9) | 15.4 (7.70) | 13.9 (7.72) |
| Median | 21.0 | 39.4 | 14.0 | 14.9 | 21.0 | 12.2 |
| Cohort 2, 9 mg/kg i.v. | | | | | | |
| Mean (SD) | 25.2 (3.80) | 33.5 (5.64) | 21.0 (7.00) | 26.5 (3.43) | 21.0 (7.00) | 17.1 (3.86) |
| Median | 28.0 | 33.6 | 21.0 | 25.8 | 21.0 | 17.1 |
| Cohort 3, 18 mg/kg i.v. | | | | | | |
| Mean (SD) | 29.4 (7.70) | 35.3 (13.1) | 23.8 (6.30) | 29.8(5.71) | 19.6 (7.70) | 17.1 (1.96) |
| Median | 28.0 | 29.3 | 28.0 | 28.6 | 14.0 | 17.0 |
| Cohort 4, 2x9 mg/kg i.v. three weeks apart | | | | | | |
| Mean (SD) | 39.2 (6.30) | 42.3 (10.7) | 36.4 (7.70) | 35.9 (8.06) | 36.4 (7.70) | 23.2 (3.76) |
| Median | 42.0 | 41.2 | 42.0 | 37.5 | 42.0 | 24.0 |
| Placebo^{†} | | | | | | |
| Mean (SD) | 10.2 (12.0) | 8.51 (5.14) | 21.5 (17.3) | 15.4 (16.1) | 10.0 (12.2) | 10.7 (7.14) |
| Median | 5.00 | 7.66 | 21.0 | 10.2 | 5.00 | 13.9 |

| | | | | | | |
|---|---|---|---|---|---|---|
| *Active-dose patients for Cohorts 1-4 (n=5) †Placebo cohort, all placebo patients pooled together (n=4). Tₘₐₓ, time of the maximum depletion of Ig. | | | | | | |

### References

References cited within this application, including patents, published applications and other publications are herein incorporated by reference.

## Claims

1. A composition comprising a fusion molecule comprising:
(i) TACI extracellular domain or fragment thereof which binds BlyS; and
(ii) a human immunoglobulin-constant domain,
for use in the treatment of SLE in a patient with mild-to-moderate SLE,
wherein said dosage is from about 1 to about 10 mg/kg and said administration occurs at weekly intervals for multiple weeks after the initial dose.

2. A composition for use according to claim 1, wherein said TACI extracellular domain has a sequence comprising SEQ ID NO:1, or wherein said TACI extracellular domain is at least 50% identical to SEQ ID NO:1.

3. A composition for use according to claim 1, wherein said human immunoglobulin-constant domain has a sequence comprising SEQ ID NO:2.

4. A composition for use according to claim 1, wherein said fusion molecule is atacicept.

5. A composition for use according to claim 1, wherein said composition is administered in amount of about 1 to about 9 mg/kg.

6. A composition for use according to Claim 1, wherein said TACI extracellular domain has a sequence comprising amino acids 30-110 of SEQ ID NO:1.

7. A composition for use according to claim 5, wherein said treatment lasts between about 2 to about 52 weeks.

8. A composition for use according to any preceding claim, wherein said use further comprises co-administration to the patient of a second medicament.

9. A composition for use according to claim 8, wherein said second medicament is selected from the group consisting of: NSAIDS, anti-malarials, corticosteroids, immunosuppressives, IVIg, DHEA, and thalidomide.

10. A composition for use according to any preceding claim, wherein said composition is administered subcutaneously, orally or intravenously.

11. A composition for use according to any preceding claim, in which the patient is a human.

12. A composition for use according to claim 4 wherein said administration is subcutaneous, and said dosage is 1 mg/kg.

13. A composition for use according to claim 4 wherein said administration is subcutaneous, and said dosage is 3 mg/kg.

14. A composition for use according to Claim 12 or 13, wherein said administration occurs at weekly intervals for four weeks after the initial dose.

## Patentansprüche

1. Zusammensetzung, umfassend ein Fusionsmolekül, das Folgendes umfasst:
(i) eine extrazelluläre TACI-Domäne oder ein Fragment davon, die/das BlyS bindet; und
(ii) eine humane Immunglobulin-konstante Domäne,
zur Verwendung bei der Behandlung von SLE bei einem Patienten mit einem milden bis mittelgradigen SLE,
wobei die genannte Dosierung von ca. 1 mg/kg bis ca. 10 mg/kg beträgt und die genannte Verabreichung in wöchentlichen Intervallen für mehrere Wochen nach der Initialdosis vorgenommen wird.

2. Zusammensetzung zur Verwendung nach Anspruch 1, wobei die genannte extrazelluläre TACI-Domäne eine Sequenz aufweist, die SEQ ID NO: 1 umfasst, oder wobei die genannte extrazelluläre TACI-Domäne zu mindestens 50 % identisch mit SEQ ID NO: 1 ist.

3. Zusammensetzung zur Verwendung nach Anspruch 1, wobei die genannte humane Immunglobulin-konstante Domäne eine Sequenz aufweist, die SEQ ID NO: 2 umfasst.

4. Zusammensetzung zur Verwendung nach Anspruch 1, wobei das genannte Fusionsmolekül Atacicept ist.

5. Zusammensetzung zur Verwendung nach Anspruch 1, wobei die genannte Zusammensetzung in einer Menge von ca. 1 mg/kg bis ca. 9 mg/kg verabreicht wird.

6. Zusammensetzung zur Verwendung nach Anspruch 1, wobei die genannte extrazelluläre TACI-Domäne eine Sequenz aufweist, welche die Aminosäuren 30 bis 110 von SEQ ID NO: 1 umfasst.

7. Zusammensetzung zur Verwendung nach Anspruch 5, wobei die genannte Behandlung zwischen ca. 2 Wochen bis ca. 52 Wochen dauert.

8. Zusammensetzung zur Verwendung nach einem der vorangehenden Ansprüche, wobei die genannte Verwendung weiter die gleichzeitige Verabreichung eines zweiten Arzneimittels an den Patienten umfasst.

9. Zusammensetzung zur Verwendung nach Anspruch 8, wobei das genannte zweite Arzneimittel aus der Gruppe ausgewählt ist, bestehend aus NSAR (nicht-steroidalen Antirheumatika (*engl.* NSAIDs, non-steroidal anti-inflammatory drugs), Antimalariamitteln, Corticosteroiden, Immunsuppressiva, IVIg, DHEA und Thalidomid.

10. Zusammensetzung zur Verwendung nach einem der vorangehenden Ansprüche, wobei die genannte Zusammensetzung subkutan, oral oder intravenös verabreicht wird.

11. Zusammensetzung zur Verwendung nach einem der vorangehenden Ansprüche, wobei der Patient ein Mensch ist.

12. Zusammensetzung zur Verwendung nach Anspruch 4 wobei die genannte Verabreichung subkutan erfolgt und die genannte Dosierung 1 mg/kg beträgt.

13. Zusammensetzung zur Verwendung nach Anspruch 4, wobei die genannte Verabreichung subkutan erfolgt und die genannte Dosierung 3 mg/kg beträgt.

14. Zusammensetzung zur Verwendung nach Anspruch 12 oder 13, wobei die genannte Verabreichung in wöchentlichen Intervallen für 4 Wochen nach der Initialdosis erfolgt.

## Revendications

1. Composition comprenant une molécule de fusion comprenant :
(i) un domaine extracellulaire de TACI ou un fragment de celui-ci qui se lie à BlyS ; et
(ii) un domaine constant d'une immunoglobuline humaine,
destinée à une utilisation dans le traitement du lupus érythémateux disséminé (LED) chez un patient atteint d'un LED léger à modéré,
ladite dose étant d'environ 1 à environ 10 mg/kg et ladite administration ayant lieu à intervalles hebdomadaires pendant plusieurs semaines après la dose initiale.

2. Composition destinée à une utilisation selon la revendication 1, dans laquelle ledit domaine extracellulaire de TACI a une séquence comprenant la SÉQ. ID n° 1, ou dans laquelle ledit domaine extracellulaire de TACI est au moins 50 % identique à la SÉQ. ID n° 1.

3. Composition destinée à une utilisation selon la revendication 1, dans laquelle ledit domaine constant d'immunoglobuline humaine a une séquence comprenant la SÉQ. ID n° 2.

4. Composition destinée à une utilisation selon la revendication 1, dans laquelle ladite molécule de fusion est l'atacicept.

5. Composition destinée à une utilisation selon la revendication 1, ladite composition étant administrée dans une quantité d'environ 1 à environ 9 mg/kg.

6. Composition destinée à une utilisation selon la revendication 1, dans laquelle ledit domaine extracellulaire de TACI a une séquence comprenant les acides aminés 30 à 110 de la SÉQ. ID n° 1.

7. Composition destinée à une utilisation selon la revendication 5, dans laquelle ledit traitement dure d'environ 2 à environ 52 semaines.

8. Composition destinée à une utilisation selon l'une quelconque des revendications précédentes, dans laquelle ladite utilisation comprend en outre la co-administration au patient d'un deuxième médicament.

9. Composition destinée à une utilisation selon la revendication 8, dans laquelle ledit deuxième médicament est sélectionné dans le groupe constitué des AINS, des anti-malariaux, des corticostéroïdes, des immunosuppresseurs, de l'Ig IV, de la DHEA, et du thalidomide.

10. Composition destinée à une utilisation selon l'une quelconque des revendications précédentes, ladite composition étant administrée par voie sous-cutanée, par voie orale ou par voie intraveineuse.

11. Composition destinée à une utilisation selon l'une quelconque des revendications précédentes, dans laquelle le patient est un humain.

12. Composition destinée à une utilisation selon la revendication 4, ladite administration étant par voie sous-cutanée, et ledit dosage étant de 1 mg/kg.

13. Composition destinée à une utilisation selon la revendication 4, ladite administration étant par voie sous-cutanée, et ledit dosage étant de 3 mg/kg.

14. Composition destinée à une utilisation selon la revendication 12 ou 13, ladite administration ayant lieu à intervalles hebdomadaires pendant quatre semaines après la dose initiale.


### SEQUENCE LISTING

<110> ZymoGenetics, Inc. Busby, Sharon Gross, Jane Visich, Jennifer Nestorov, Ivan Munafo, Alain Papasouliotis, Orestis Pena Rossi, Claudia
<120> DOSING METHODS FOR TREATING AUTOIMMUNE DISEASES USING A TACI-IG FUSION MOLECULE SUCH AS ATACICEPT
<130> 07-15PC
<150> 60/943,618
   <151> 2007-06-13
<150> 61/024,031
   <151> 2008-01-28
<160> 2
<170> PatentIn version 3.4
<210> 1
   <211> 154
   <212> PRT
   <213> TACI
<400> 1
   Met Ser Gly Leu Gly Arg Ser Arg Arg Gly Gly Arg Ser Arg Val Asp
   1               5                   10                  15
   Gln Glu Glu Arg Phe Pro Gln Gly Leu Trp Thr Gly Val Ala Met Arg
               20                  25                  30
   Ser Cys Pro Glu Glu Gln Tyr Trp Asp Pro Leu Leu Gly Thr Cys Met
           35                  40                  45
   Ser Cys Lys Thr Ile Cys Asn His Gln Ser Gln Arg Thr Cys Ala Ala
       50                  55                  60
   Phe Cys Arg Ser Leu Ser Cys Arg Lys Glu Gln Gly Lys Phe Tyr Asp
   65                  70                  75                  80
   His Leu Leu Arg Asp Cys Ile Ser Cys Ala Ser Ile Cys Gly Gln His
                   85                  90                  95
   Pro Lys Gln Cys Ala Tyr Phe Cys Glu Asn Lys Leu Arg Ser Pro Val
               100                 105                 110
   Asn Leu Pro Pro Glu Leu Arg Arg Gln Arg Ser Gly Glu Val Glu Asn
           115                 120                 125
   Asn Ser Asp Asn Ser Gly Arg Tyr Gln Gly Leu Glu His Arg Gly Ser
       130                 135                 140
   Glu Ala Ser Pro Ala Leu Pro Gly Leu Lys
   145                 150
<210> 2
   <211> 348
   <212> PRT
   <213> Human Immunoglobulin-Constant Domain Fc5
<400> 2
   Met Asp Ala Met Lys Arg Gly Leu Cys Cys Val Leu Leu Leu Cys Gly
   1               5                   10                  15
   Ala Val Phe Val Ser Leu Ser Gln Glu Ile His Ala Glu Leu Arg Arg
               20                  25                  30
   Phe Arg Arg Ala Met Arg Ser Cys Pro Glu Glu Gln Tyr Trp Asp Pro
           35                  40                  45
   Leu Leu Gly Thr Cys Met Ser Cys Lys Thr Ile Cys Asn His Gln Ser
       50                  55                  60
   Gln Arg Thr Cys Ala Ala Phe Cys Arg Ser Leu Ser Cys Arg Lys Glu
   65                  70                  75                  80
   Gln Gly Lys Phe Tyr Asp His Leu Leu Arg Asp Cys Ile Ser Cys Ala
                   85                  90                  95
   Ser Ile Cys Gly Gln His Pro Lys Gln Cys Ala Tyr Phe Cys Glu Asn
               100                 105                 110
   Lys Leu Arg Ser Glu Pro Lys Ser Ser Asp Lys Thr His Thr Cys Pro
           115                 120                 125
   Pro Cys Pro Ala Pro Glu Ala Glu Gly Ala Pro Ser Val Phe Leu Phe
       130                 135                 140
   Pro Pro Lys Pro Lys Asp Thr Leu Met Ile Ser Arg Thr Pro Glu Val
   145                 150                 155                 160
   Thr Cys Val Val Val Asp Val Ser His Glu Asp Pro Glu Val Lys Phe
                   165                 170                 175
   Asn Trp Tyr Val Asp Gly Val Glu Val His Asn Ala Lys Thr Lys Pro
               180                 185                 190
   Arg Glu Glu Gln Tyr Asn Ser Thr Tyr Arg Val Val Ser Val Leu Thr
           195                 200                 205
   Val Leu His Gln Asp Trp Leu Asn Gly Lys Glu Tyr Lys Cys Lys Val
       210                 215                 220
   Ser Asn Lys Ala Leu Pro Ser Ser Ile Glu Lys Thr Ile Ser Lys Ala
   225                 230                 235                 240
   Lys Gly Gln Pro Arg Glu Pro Gln Val Tyr Thr Leu Pro Pro Ser Arg
                   245                 250                 255
   Asp Glu Leu Thr Lys Asn Gln Val Ser Leu Thr Cys Leu Val Lys Gly
               260                 265                 270
   Phe Tyr Pro Ser Asp Ile Ala Val Glu Trp Glu Ser Asn Gly Gln Pro
           275                 280                 285
   Glu Asn Asn Tyr Lys Thr Thr Pro Pro Val Leu Asp Ser Asp Gly Ser
       290                 295                 300
   Phe Phe Leu Tyr Ser Lys Leu Thr Val Asp Lys Ser Arg Trp Gln Gln
   305                 310                 315                 320
   Gly Asn Val Phe Ser Cys Ser Val Met His Glu Ala Leu His Asn His
                   325                 330                 335
   Tyr Thr Gln Lys Ser Leu Ser Leu Ser Pro Gly Lys
               340                 345